(19) Europäisches Patentamt / European Patent Office / Office européen des brevets

(11) **EP 4 241 761 A1**

(12) **EUROPEAN PATENT APPLICATION**
published in accordance with Art. 153(4) EPC

(43) Date of publication:
**13.09.2023 Bulletin 2023/37**

(21) Application number: **21889587.8**

(22) Date of filing: **04.11.2021**

(51) International Patent Classification (IPC):
*A61K 9/10* (2006.01)  *A61K 47/02* (2006.01)
*A61K 47/40* (2006.01)  *A61K 47/38* (2006.01)
*A61K 47/18* (2017.01)  *A61K 47/12* (2006.01)
*A61K 47/26* (2006.01)  *A61K 31/404* (2006.01)
*A61K 31/4439* (2006.01)  *A61K 31/506* (2006.01)

(52) Cooperative Patent Classification (CPC):
A61K 9/10; A61K 31/404; A61K 31/4439;
A61K 31/506; A61K 47/02; A61K 47/12;
A61K 47/18; A61K 47/26; A61K 47/38; A61K 47/40

(86) International application number:
**PCT/KR2021/015913**

(87) International publication number:
**WO 2022/098122 (12.05.2022 Gazette 2022/19)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **09.11.2020 KR 20200148851**
**23.08.2021 KR 20210110809**

(71) Applicant: **Scai Therapeutics Co., Ltd.**
**Seoul 06524 (KR)**

(72) Inventors:
• **KIM, Chulhwan**
**Daejeon 35249 (KR)**
• **KIM, Kyoung-hee**
**Daejeon 35249 (KR)**
• **SUH, Daewoong**
**Seoul 06288 (KR)**

(74) Representative: **dompatent von Kreisler Selting Werner -
Partnerschaft von Patent- und Rechtsanwälten mbB
Deichmannhaus am Dom
Bahnhofsvorplatz 1
50667 Köln (DE)**

(54) **SOLID CYCLOSPORIN A AND DISPERSION COMPOSITION COMPRISING SAME**

(57) Provided is a dispersion composition comprising: a dispersion medium; and particles comprising a target substance, wherein the dispersion composition comprises at least one type of surfactant having a critical micelle concentration or more and does not comprise a solubilizer, and the target substance is cyclosporin A, which satisfies the S-parameter > 1 defined above.

## Description

[Technical Field]

The present invention relates to a solid cyclosporin A having improved dispersibility and a dispersion composition obtained by dispersing the same, which exhibits improved dispersibility.

[Background Art]

In the field of pharmaceutical technology, preparing an aqueous solution composition is one of the important tasks. Water is the most common solvent and liquid phase for drinking, which is the most used solvent for drug formulations. However, many drugs are strongly non-polar, and thus have insufficient solubility in water which is a polar solvent. As such, despite the excellent therapeutic efficacy, insoluble or poorly-soluble drugs with poor solubility have a problem in that they cannot be made into clinically useful pharmaceutical formulations.

As a representative known technology to solve these problems, there is micelle solubilization technology that solubilizes poorly soluble drugs by surfactant. The surfactant is a substance that has both hydrophilicity and hydrophobicity in one molecule, which generally refers to substances that can help disperse the target substance or stabilize the dispersion state, or substances that perform these functions, by being mainly distributed at the interface of the target substance (e.g., drug) and the dispersion medium (e.g., water). The micelle solubilization technology is a technology for containing a target substance in a dispersion medium at a high content exceeding saturation solubility, by selectively distributing the poorly soluble target substance in the dispersion medium to the nanometer-sized micelle formed by self-assembly of the surfactant contained in the dispersion medium in excess of a specific concentration (critical micelle concentration).

[Disclosure]

[Technical Problem]

In pharmaceutical technology, the bioavailability is a concept representing the amount and rate at which a drug is delivered from the site of administration to the site of action in vivo where the drug causes a therapeutic effect, and if the bioavailability is low, even if the drug has high therapeutic efficacy in vitro, the actual amount delivered to the in vivo organs where it should act is low, and thus the therapeutic effect in the body remains at a low or insignificant level. The bioavailability can be understood as the FLUX through a biological barrier in vivo, such as the cornea, the skin, the blood-brain-barrier, and the blood-retina barrier (see Equation 8 below).

$$[\text{Equation 8}]$$
$$\text{FLUX} = \text{solubility} * \text{permeability}$$

wherein it can be seen that in order to increase the FLUX, both solubility and permeability must be high in balance. According to The Biopharmaceutics Classification System, 70% of all drugs belong to Class II with high permeability and low solubility, and 20% belong to Class IV with low permeability and low solubility. (Reintjes, T., Solubility enhancement with BASF Pharma polymers: Solubilizer Compendium. BASF, (2021) pp.9-10.).

If the micelle solubilization technology is applied to a polar dispersion medium such as water, the outer surface of the drug surrounded by the surfactant becomes hydrophilic compared to the state in which the drug exists independently. However, since most of the physiological barriers in vivo include a phospholipid layer or a hydrophobic layer, the permeability of the biological barrier of the pharmaceutical composition implemented by the micelle solubilization technology is generally lowered. For this reason, the micelle solubilization technology has a trade-off between improving solubility and increasing permeability, and thus has a limitation in that it is difficult to obtain a pharmaceutical composition with improved bioavailability to the extent that therapeutic efficacy is improved. In addition, most surfactants have problems such as causing environmental pollution or causing toxicity or side effects in the body, and thus there is a need to minimize the amount of surfactant used.

Therefore, it can be said that the necessity and commercial value of technologies that can achieve improvement in solubility even if the amount of surfactant used is lowered or minimized than existing technologies is very great. These technologies can improve permeability compared to existing technologies by lowering the amount of surfactant required than the existing technologies, and as a result, can improve bioavailability or improve treatment efficacy, and can also minimize environmental pollution or toxicity/side effects in the body.

The objects of the present invention are not limited to the above-mentioned objects, and other objects and advantages of the present invention not mentioned above can be understood by the following description and will be more clearly understood by the examples of the present invention. It will also be easily understood that the objects and advantages of the present invention may be realized by means set forth in the claims, and combinations thereof.

Cyclosporin is an immune-suppressive drugs with 11 non-polar cyclic amino acid structures and comprise cyclosporin A, B, C, D and G. In immune lymphoid cells, cyclosporin has a function of blocking the activity of calcineurin, thereby suppressing the production of immune cytokines that ultimately lead to an immune response. In addition to various immune diseases, cyclosporin is known to be effective in recovering/restoring collapse and dysfunction of the lacrimal gland in Keratoconjunctivitis sicca or Dry Eye Syndrome (US patent 4839342).

However, it is difficult to achieve the therapeutic effect of cyclosporin in ocular tissues due to its large molecular weight and hydrophobic nature. In particular, since cyclosporin is an insoluble substance, it has the disadvantage that it is difficult to expect improved solubility in aqueous solutions or aqueous media (US patent 5051402). In order to compensate for these disadvantages, many attempts have been made to solubilize cyclosporin in pharmaceutical compositions for the treatment of dry eye syndrome. For example, the cyclosporin formulations of Restasis, Cequa, and Ikervis developed by Allergan company, Sun Pharma company, and Santen company, known previously, contain surfactants such as Polysorbate 80, hydrogenated castor oil and Poloxamer (US patent 2014020662A1p, 2014005785A1p, EP2049079B1). However, a large amount of surfactant can cause side effects that cause environmental pollution and toxicity in the body.

In addition, although the cyclosporin formulations of Restasis and Ikervis were prepared as solutions containing Castor oil and MCT oil (Medium Chain Triglycerides), respectively, ocular compositions containing oils can cause side effects such as blurred vision and eye irritation when dropped in the eye.

In order to minimize these side effects and improve bioavailability, it is necessary to minimize the content of surfactant or oil such as castor oil. Therefore, there is still a need to develop a cyclosporin formulation that uses a smaller amount of surfactant than the known technology, but has improved solubility in a polar dispersion medium.

[Technical Solution]

In one embodiment of the present invention, the present invention provides a dispersion composition comprising a dispersion medium; and particles comprising a target substance,

wherein the dispersion composition comprises at least one type of surfactant having a critical micelle concentration or more,
the dispersion composition does not comprise a solubilizer,
the target substance is cyclosporin A,
if the dispersion composition comprises at least one type of surfactant, the S-parameter of Equation 3 calculated by Equation 1 and Equation 2 satisfies S-parameter > 1, and
if the dispersion composition comprises at least two types of surfactants having a critical micelle concentration or more, $S_{surf(i)}$ obtained by Equation 4 below is calculated for each type of surfactant, and then the $S_{surf}$ value is obtained as the sum of these by Equation 5 below and the S-parameter of Equation 3 obtained by applying the calculated $S_{surf}$ value to Equation 1 above satisfies S-parameter > 1.

$$<\text{Equation 1}>$$

$$S_{micelle} = S_w + S_{surf}$$

wherein $S_w$ is the concentration corresponding to the saturation solubility of the target substance in the dispersion medium, and $S_{surf}$ is calculated by Equation 2 below.

$$<\text{Equation 2}>$$

$$S_{surf} = k(C_{surf} - CMC)$$

wherein k is the molar solubilization capacity defined as the number of moles of the target substance that can be dispersed in the dispersion medium by one type of surfactant having the critical micelle concentration of 1 mole or more, and $C_{surf}$ is the molar concentration of the surfactant component in the composition, and CMC is the critical micelle molar concentration of the surfactant in the composition.

$$\text{<Equation 3>}$$

$$\text{S-parameter} = S_{tot} / S_{micelle}$$

wherein $S_{tot}$ is the total molar content of the target substance contained in the dispersion composition.

$$\text{<Equation 4>}$$

$$S_{surf(i)} = k_{surf(i)} (C_{surf(i)} - CMC_{surf(i)})$$

wherein $k_{surf(i)}$ is a molar solubilization capacity defined as the number of moles of the target substance that can be dispersed in the dispersion medium by any one type of the surfactant components having the critical micelle concentration of 1 mole or more, $C_{surf(i)}$ is the concentration of any one type of the surfactant components having the critical micelle concentration or more, and $CMC_{surf(i)}$ is the critical micelle concentration in the dispersion medium of any one type of the surfactant components having the critical micelle concentration or more.

$$\text{<Equation 5>}$$

$$S_{surf} = \sum_{i=1}^{m} S_{surf(i)}$$

wherein m is the total number of types of surfactant components having the critical micelle concentration or more.

In another embodiment of the present invention, the present invention provides solid cyclosporin A that can be applied as the target substance. That is, the dispersion composition is obtained by applying the solid cyclosporin A as the target substance.

[Advantageous Effects]

In the fields of dispersion compositions such as drugs and cosmetics, stably dispersing a high content of active substances is an important task for easy absorption of active substances. Therefore, the dispersion composition can be innovatively applied to various industrial fields requiring high content and dispersion stability. For example, if the dispersion composition implemented by the present invention contains poorly soluble drugs, it is possible to expand the availability of previously unavailable drugs and to minimize the side effects of additives such as surfactants, thereby achieving significant advances in disease treatment.

The dispersion composition implemented by the present invention has a lower content of surfactant than those of existing technologies, and improves not only the solubility of the target substance but also the permeability, thereby resulting in improved bioavailability and excellent therapeutic efficacy. In addition, by lowering the content of surfactants compared to existing technologies, there is an effect of reducing or preventing problems such as environmental pollution or toxicity/side effects in the body caused by the surfactants. For example, Polyoxyl 35 castor oil (Kolliphor EL or Cremophor EL), which is mainly used as a surfactant in pharmaceutical compositions, is highly toxic. In addition, even when preparing the dispersion of cyclosporin A, Polysorbate 80 (Polysorbate 80 or Tween 80), which is a surfactant, is used, which has the disadvantage of severe eye irritation. The dispersion composition of the present invention can reduce or avoid the side effects of toxic surfactants because the surfactant content is low and a stable dispersion state is maintained. In another example, a cosmetic product made of the dispersion composition embodied in the present invention is a composition that minimizes the content of additives such as surfactant, and can improve cosmetic effects by improving spreadability and penetrability.

In addition to the above effects, specific effects of the present invention will be described together while explaining specific details for carrying out the present invention.

[Best Mode]

<Definition of Terms>

First, definitions/descriptions of key terms used in the description of the present invention are as follows.

The term "target substance" means a substance to be dispersed in a dispersion composition to be implemented, which may be an active pharmaceutical substance for pharmaceutical use, an active pharmaceutical substance effective for skin aesthetics for cosmetics, and various pharmaceutical substances depending on use. For example, the pharmacologically active pharmaceutical substance in the present invention may be, but is not limited to, tyrosine kinase inhibitors such as Sunitinib, Axitinib and Pazopanib, cyclosporine, Niclosamide, Adenosine, Deoxycholic acid, Paclitaxel or pharmaceutically acceptable salts or derivatives thereof. The target substance may be one type of pharmaceutical substance or a mixture of two or more types of target substances. The dispersion composition according to one embodiment of the present invention is commercially valuable when the target substance is a poorly soluble pharmaceutical substance in the dispersion medium. Examples of drugs that are poorly soluble pharmaceutical substances may comprise substances classified as pi (practically insoluble), vss (very slightly soluble), ss (slightly soluble), sps (sparingly soluble), etc. in the "United States Pharmacopeia (USP) Solubility Criteria" (O. Wolk, et al, Drug Design, Development and Therapy, 8 (2014) pp 1563-1575). Examples of drugs as the target substance may comprise, but are not limited to, drugs that are insoluble or poorly soluble in water, such as Paclitaxel, Deoxycholic acid, cyclosporine, Minoxidil, Finasteride, Latanoprost, Miconazole, Prednisolone, Fluorometholone or prostaglandin analogs, or pharmaceutically acceptable salts of these drugs or derivatives of these drugs, or combinations of these. In addition, the target substance may be a nutritional component or an active substance having a cosmetic effect that is poorly soluble in water, such as curcumin, and may be various substances depending on the use or purpose of the dispersion composition.

The term "medium with a plurality of surfaces" means a medium composed of porous materials or non-porous materials or mixtures thereof, and containing intraparticle pores or interparticle pores or a mixture thereof. That is, the pores in the medium with a plurality of surfaces may be intraparticle pores or interparticle pores. If the medium with a plurality of surfaces is, for example, a porous material containing pores inside the material, the pores in the medium with a plurality of surfaces may be intraparticle pores contained in the porous material. For example, if the medium with a plurality of surfaces is formed as an aggregate or agglomerate (secondary particles, powder or packed-bed, etc.) formed by aggregating or stacking non-porous particles, the pores within the medium with a plurality of surfaces may be interparticle pores. For example, if the medium with a plurality of surfaces is formed as an aggregate or agglomerate (secondary particles, powder or packed-bed, etc.) formed by mixing porous particles and non-porous particles and aggregating or stacking them, the pores within the medium with a plurality of surfaces may be intraparticle pores or interparticle pores. That is, the medium with a plurality of surfaces may be an aggregate or agglomerate composed of various types of porous materials, or arbitrary porous or non-porous particles.

An average size of the medium with a plurality of surface pores may be about 1 nm to about 1 gm. For example, the average size of the medium with a plurality of surface pores may be about 1 nm to about 100 nm. For example, the average size of the medium with a plurality of surface pores may be about 1 nm to about 50 nm. For example, the average size of the medium with a plurality of surface pores may be about 1 nm to about 30 nm. By using a medium with a plurality of surfaces having a pore size in the above range, it may help to form particles with a predetermined desired size. The porosity of the medium with a plurality of surfaces may be about 5 to about 97% (v/v). For example, the porosity of the medium with a plurality of surfaces may be about 20 to about 60% (v/v). For example, when the medium with a plurality of surfaces is aerogel, the porosity may be about 90 to about 97% (v/v).

For example, the medium with a plurality of surfaces may be silica gel, silica xerogel, mesoporous silica, fumed Silica, mesoporous alumina, mesoporous metal oxides, mesoporous material, charcoal, activated carbon, aerogel, zeolite, molecular sieve, metal-organic framework, organic/inorganic hybrid porous materials, and may be natural materials, synthetic materials, or biological materials, and may be crystalline or amorphous, and is not limited by composition, material, structure, synthesis/manufacturing method, and the like. The pores in the medium with a plurality of surfaces may have various shapes, sizes, generation methods, and arrangement structures (regular or irregular). The medium with a plurality of surfaces may be the form of particles having an arbitrary size and shape, the form of secondary particles in which a plurality of particles are gathered, the form of powder composed of particles having an arbitrary size and shape, the form of a packed-bed formed by stacking particles, the form of solid foam, or the form of a membrane or sheet, but is not limited by the form. In addition, the medium with a plurality of surfaces may comprise not only a single material but also at least two or more types of materials, or may be a mixture thereof.

That is, if the size, shape, porosity, predetermined surface area, or surface physico-chemical properties of the intraparticle or interparticle pore are suitable for the use of the method and composition of the present invention, the medium with a plurality of surfaces is not limited in the material, phase, crystalline or amorphous state, composition, size, shape, form, formation method or aggregation method, and void arrangement structure of the medium with a plurality of surfaces, or is not limited to whether the pores of the medium with a plurality of surfaces are intra-particle or interparticle pores, and whether the medium with a plurality of surfaces is porous materials or non-porous materials.

The term "solvent for producing mixed liquor" means a solvent capable of dissolving the target substance in the intended content, which may be selected in consideration of physical properties of the solvent such as saturation solubility or polarity so as to dissolve the target substance in a target content. The target substance may be dissolved in a higher content in the solvent for preparing the mixed liquor than in the dispersion medium. For example, the solubility of the target substance in the solvent for preparing the mixed liquor is greater than the solubility of the target substance in a dispersion medium. For example, the solvent for preparing the mixed liquor may comprise water, organic solvents such as alcohol (methanol, ethanol, etc.), acetone, acetic acid, acetonitrile, ethyl acetate, methylene chloride, chloroform and dimethyl sulfoxide (DMSO), sugars such as polyethylene glycol (PEG), mannitol and sorbitol, and may comprise a combination of two or more thereof, but is not limited thereto. The solvent for preparing the mixed liquor may be friendly or non-friendly to the dispersion medium. The solvent for preparing the mixed liquor may be miscible, immiscible, or incompletely miscible with the dispersion medium. The solvent for preparing the mixed liquor may be a hydrophilic material, a hydrophobic material, or an amphiphilic material. The solvent for preparing the mixed liquor may be polar, non-polar or amphiphilic. The solvent for preparing the mixed liquor may be a volatile compound. When the dispersion composition is prepared by finally removing the solvent for preparing the mixed liquor, if the solvent for preparing the mixed liquor is a volatile compound, there is an advantage that the solvent for preparing the mixed liquor is easily removed. The solvent for preparing the mixed liquor may be a compound having a lower boiling point than the dispersion medium. When the dispersion composition is prepared by finally removing the solvent for preparing the mixed liquor, if the solvent for preparing the mixed liquor is a compound having a lower boiling point than the dispersion medium, there is an advantage that the solvent for preparing the mixed liquor is easily removed by distillation, which is one of the separation processes.

The term "mixed liquor" means a solution obtained by mixing the target substance with the solvent for preparing the mixed liquor so that the target substance is dissolved in the solvent for preparing the mixed liquor. For example, the mixed liquor may comprise the target substance in an amount of about 0.01% (w/v) to about 50% (w/v). Specifically, the mixed liquor (A) may comprise the target substance in an amount of about 0.1% (w/v) to about 10% (w/v). The content of the target substance in the mixed liquor may be determined by considering the solubility of the target substance in the solvent for preparing the mixed liquor, and accordingly, the type of solvent for preparing the mixed liquor can be selected.

The term "process fluid" means a fluid used with the mixed liquor when contacting the medium with a plurality of surfaces, for the purpose of easiness of contact, increase in productivity, or control of retention time of mixed liquor in a medium with a plurality of surfaces when contacting the mixed liquor with the medium with a plurality of surfaces. The process fluid is selected in consideration of viscosity or surface tension in order to adjust the ease of contact, productivity or retention time of the mixed liquor, and the type of the process fluid may be selected by appropriately considering the solubility of the target substance or the miscibility with the solvent for preparing mixed liquor so that the target substance does not precipitate or solidify in the process of contact between the mixed liquor and the medium with a plurality of surfaces. The process fluid may be the same as or different from the solvent for preparing the mixed liquor, or may be a mixture with a fluid different from the solvent for preparing the mixed liquor.

The term "dispersion medium" corresponds to the continuous phase among the components of the dispersion composition, and various substances may be used depending on the application. For example, the dispersion medium, if for a pharmaceutical composition, may be water, a saline solution or a buffered aqueous solution. In the dispersion composition of the present invention, the content of the target substance may exceed saturation solubility in the dispersion medium.

The dispersion medium may be a polar or hydrophilic solvent. For example, the polar or hydrophilic solvent may be water, methanol, ethanol, glycerol, or polyol. For example, the dispersion medium may be water, and the target substance may be water-insoluble paclitaxel, deoxycholic acid, cyclosporin, latanoprost, miconazole, curcumin, or the like. The dispersion medium may be a non-polar or hydrophobic solvent. For example, the non-polar or hydrophobic solvent may be hydrocarbon, silicone oil, ethyl acetate, acetone, tetrahydrofuran (THF) or the like. For example, the dispersion medium may be hexane, and the target substance may be hexane-insoluble saccharides, glucose or the like. In addition, the dispersion medium may be an amphiphilic solvent. In addition, the dispersion medium may be a polar or non-polar solvent to which an amphiphilic solvent is added. The type or composition of the dispersion medium may be selected considering variously the solubility of the target substance in it, the difference in physico-chemical physical properties, the use of applying the dispersion composition and the like, and one type or a mixture of two or more types may be used. For example, the dispersion medium may be an organic solvent.

The term "particle" is defined as an association of multiple molecules having an arbitrary composition, shape, size, or structure, and corresponds to a dispersed phase, which is a discrete phase in a dispersion composition. In the present invention, particles in a dispersion composition may further comprise an auxiliary agent or an additive substance in addition to the target substance.

The term "dispersion composition" means a composition in which particles comprising the target substance are dispersed in a dispersion medium, which is a continuous phase, as a discrete phase distinct from the dispersion medium. The dispersion composition is a state distinct from a single-phase solution in which the target substance is dissolved in a solvent as a solute because the particles comprising the target substance are dispersed in the dispersion medium as a different phase distinct from the dispersion medium, while an interface (or interphase boundary) is formed between the particles and the dispersion medium. The dispersion composition may additionally comprise additives or auxiliary agents for adjusting the physical properties and quality required according to the intended use or administration route, such as viscosity, osmotic pressure, pH, ionic strength, surface tension, color, taste, and fragrance.

The term "surfactant" means a surface active and amphiphilic material that structurally possesses both a hydrophilic head and a hydrophobic tail in a molecule and lowers surface tension or interfacial tension in a dispersion medium, and generally refers to a material that is added to the dispersion composition in a smaller amount than the dispersion medium and can help disperse the target substance or stabilize the dispersion state by being mainly distributed at the interface between the target substance and the dispersion medium, or a substance that performs these functions. The surfactant is also referred to as an emulsifier or detergent depending on its use or industry. The surfactant may be a monomer, oligomer or polymer, and may have various molecular weights. In addition, the surfactant can be cationic, anionic, nonionic or zwitterionic, and the ionic state can be changed by pH. In addition, the surfactant may be a natural material, synthetic material, or biological material, and a mixture of a plurality of materials may be used, but is not limited thereto.

The term "critical micelle concentration (CMC)" means the concentration at which the surfactant added to the dispersion medium is self-assembled to form particles (hereinafter collectively referred to as micelle) having a colloidal size (1 nm - 1 um). If the surfactant is less than the critical micelle concentration, the solubility of the target substance in the dispersion medium has a saturated solubility of the target substance (the saturation solubility, that is, the saturation solubility of a specific target substance in a specific dispersion medium is determined as a unique value depending on conditions comprising the type, composition, phase and content of the target substance, the type and composition of the dispersion medium, and temperature and pressure. For example, the saturation solubility of the target substance in the dispersion medium is described in "The Merck Index: An Encyclopedia of Chemicals, Drugs and Biologicals" or PubChem: Open Chemistry Database at the National Institutes of Health (NIH) which is a well-known database), but it is well known that if the concentration of surfactant added to the dispersion medium exceeds the critical micelle concentration, the behavior (micelle solubilization, solubilization by micelle or micellar solubilization) increases in proportion to the amount of micelle (M.J. Rosen, J.T. Kunjappu, Surfactants and Interfacial Phenomena, Fourth edition, 2012, Wiley). For example, the reference literature, M. J. Rosen & J.T. Kunjappu, "Surfactants and Interfacial Phenomena", 4th Ed, Wiley (2012), pp141-143, p155 lists the critical micelle concentration values of various surfactants.

The term "molar solubilization capacity ($\kappa$)" is the number of moles of target substance solubilized in the dispersion medium per mole of surfactant exceeding the critical micelle concentration (CMC), which is defined by Equation 9 below and usually has a value less than 1. In Equation 9 below, $S_w$ is the mole saturation solubility, which is a unique value of the target substance in the dispersion medium, $S_{tot}$ represents the total molar content of the target substance contained in the dispersion composition, and $C_{surf}$ represents the total molar content of the surfactant contained in the dispersion composition (Rangel-Yagui CO, Pessoa A Jr, Tavares LC., J Pharm Pharm Sci. 2005 8(2):147-65.). When the content of the target substance (y-axis) is measured and graphed according to the surfactant content (x-axis), if the surfactant is not contained or its content is less than CMC, the content of the target substance is saturated solubility or does not deviate greatly from saturation solubility. When the surfactant starts to exceed the CMC, the content of the target substance shows a linearly increasing behavior in proportion to the content of the surfactant (micelle solubilization, solubilization by micelle or micellar solubilization). The molar solubilization capacity ($\kappa$) corresponds to the slope of the straight line section in the above graph and is an index indicating the amount of target substance that can be solubilized by a unit amount of surfactant, and the surfactant with high molar solubilization capacity ($\kappa$) means high solubilization efficiency. The molar solubilization capacity ($\kappa$) corresponds to physical properties that have unique values depending on the surfactant, target substance, and dispersion medium.

[Equation 9]

$$\kappa = \frac{S_{tot} - S_w}{C_{surf} - CMC}$$

The term "solubilizer" generally refers to a substance that is added in a smaller amount than the dispersion medium to the dispersion composition in which the target substance is dispersed in the dispersion medium, and serves to increase the content of the target substance to a content exceeding the saturation solubility of the target substance in the dispersion medium, which is a substance distinct from the target substance and dispersion medium. The solubilizer may be, for example, cyclodextrin, liposome, oil, liquid, solid or nanostructured lipid, metallic/organic/inorganic nanoparticle, porous medium, a water-soluble polymer, an antibody, or the like, and may comprise a combination of two or more thereof, but is not limited thereto. The target substance may be contained in an amount exceeding the saturation solubility in the dispersion medium through the solubilizer and various physico-chemical mechanisms. These physico-chemical mechanisms comprise complexation, inclusion, encapsulation, conjugation, adsorption, absorption, dissolution, and the like, but are not limited thereto. The surfactant has surface activity (physical properties that reduce the surface/interfacial tension of the dispersion medium), and shows a characteristic concentration called critical micelle concentration in the dispersion medium, and the surfactant self-assembles to form micelle when the concentration exceeds the critical micelle concentration in the dispersion medium, and as the micelle is formed, the physico-chemical physical properties (osmotic pressure, turbidity, diffusion coefficient, surface tension, electrical conductivity, etc.) become different from before the micelle formation, and the target substance is selectively distributed in the formed micelle, and thus the surfactant is clearly distinguished from other solubilizers in mechanism or physical properties, and in the present invention, it is treated as a separate substance from solubilizer and is referred to separately from solubilizer.

In this specification, unless otherwise specified, terminology follows the definitions and recommendations of the International Union of Pure and Applied Chemistry (IUPAC).

Hereinafter, embodiments of the present invention will be described in detail. However, these embodiments are presented as examples, and the present invention is not limited thereto, and the present invention is only defined by the scope of the claims to be described later.

The dispersion composition according to one embodiment of the present invention provides a dispersion composition containing a target substance stably dispersed in a dispersion medium in excess of the maximum amount ($S_{micelle}$) that can be solubilized by micelle solubilization technology. With the existing micelle solubilization technology, it was not possible to stably disperse the target substance in a dispersion medium with a content higher than the maximum amount ($S_{micelle}$) that can solubilize the target substance.

In one embodiment of the present invention, the present invention provides the product of the separated solid target substance obtained by removing the solvent for preparing the mixed liquor and the process fluid.

In the dispersion composition according to one embodiment of the present invention, the target substance is cyclosporin A.

In one embodiment of the present invention, the dispersion composition is a dispersion composition in which the target substance of the separated solid phase is dispersed in the dispersion medium by mixing the product of the separated solid target substance with the separately prepared dispersion medium, the surfactant, and necessary additives, and the dispersion composition is a dispersion composition containing a target substance stably dispersed in a dispersion medium in excess of the maximum amount ($S_{micelle}$) that can be solubilized by micelle solubilization technology.

One embodiment of the present invention is to provide a dispersion composition having a high target substance content (i.e., increased solubilization efficiency) while lowering the content of surfactant than the amount required in existing micelle solubilization technology.

One embodiment of the present invention provides a dispersion composition containing a target substance exceeding the content that can be solubilized by existing micelle solubilization technology.

One embodiment of the present invention provides a pharmaceutical composition with improved bioavailability or therapeutic efficacy by lowering the content of surfactant than the amount required in existing micelle solubilization technology.

The present invention is not limited to drugs, and can be equally applied to fields that increase solubility or bioavailability in vivo. For example, in the field of cosmetics, many of the substances with excellent cosmetic effect have poor solubility, and thus various additives are added to the final formulation. The cosmetic product made in this way has an opaque formulation, and thus do not have a good sense of aesthetics and do not have an excellent tactile feel to the skin. In addition, excessively added additives block pores of the skin, preventing easy absorption of active substances. As such, the present invention is not limited to a specific target substance, a specific dispersion medium, or a specific efficacy/function.

When the dispersion composition contains one type of surfactant exceeding the critical micelle concentration, the maximum content of the target substance that can be solubilized by the existing micelle solubilization technology is determined by the $S_{micelle}$ value obtained through Equations 1 and 2 below.

$$\text{<Equation 1>}$$

$$S_{micelle} = S_w + S_{surf}$$

wherein $S_w$ is the concentration corresponding to the saturation solubility of the target substance in the dispersion medium, and $S_{surf}$ is calculated by Equation 2 below.

<Equation 2>

$$S_{surf} = k(C_{surf} - CMC)$$

wherein k is the known molar solubilization capacity ($\kappa$) measured in the dispersion medium for the used surfactant and target substance, $C_{surf}$ is the molar concentration of the surfactant component added to the composition, and CMC is the critical micelle molar concentration of the known surfactant specified in the dispersion medium. $S_{surf}$ can therefore be calculated using known physical properties ($\kappa$, CMC) measured for the target substance, surfactant and dispersion medium.

The S-parameter is a value obtained by dividing the total content ($S_{tot}$) of the target substance contained in the dispersion composition according to one embodiment of the present invention by the maximum content ($S_{micelle}$) of the target substance that can be solubilized by existing micelle solubilization technology as calculated by Equation 3 below.

<Equation 3>

$$S\text{-parameter} = S_{tot} / S_{micelle}$$

When the dispersion composition implemented by the present invention contains one type of surfactant exceeding the critical micelle concentration, the content ($S_{tot}$) of the target substance that can be stably contained in the dispersion composition exceeds the value of $S_{micelle}$ (Equations 1 and 2). That is, the S-parameter of the dispersion composition containing one type of surfactant implemented by the present invention exceeds 1. For example, in the dispersion composition implemented by the present invention, the S-parameter value may be 1.05 or more, 1.06 or more, 1.1 or more, 1.2 or more, 1.5 or more, 2 or more, or 3 or more.

In the dispersion composition according to one embodiment of the present invention, which can be prepared by a method described later, the S-parameter is implemented with a value exceeding 1. In addition, in a dispersion composition prepared by using a solid target substance (e.g., which may be powder) according to an embodiment of the present invention described below, the S-parameter is implemented with a value exceeding 1.

Since $S_{micelle}$ is a value calculated using the experimentally measured physical properties of the target substance, surfactant, and dispersion medium, the content of the target substance in the dispersion composition realized by the existing micelle solubilization technology cannot exceed $S_{micelle}$, and thus the S-parameter of the dispersion composition realized by the existing micelle solubilization technology is no more than 1. However, as described above, since the dispersion composition implemented by the present invention has an S-parameter exceeding 1, the present invention can implement a dispersion composition containing a target substance having a higher content beyond the limit that can be solubilized by the existing micelle solubilization technology. Expressing these matters in terms of the surfactant, it means that the present invention can implement a dispersion composition containing the above-described specific content of the target substance, even when using a smaller amount of surfactant than the surfactant required to solubilize the specific content of the target substance by the existing micelle solubilization technology.

As described above, with the existing micelle solubilization technology, since the surfactant cannot stably disperse the target substance in the dispersion medium with a content higher than the maximum amount ($S_{micelle}$) that can solubilize the target substance, it is natural in terms of concept definition that the S-parameter for the dispersion composition prepared by incorporating the surfactant by the existing micelle solubilization technology cannot exceed 1.

However, when the S-parameter is experimentally measured for the dispersion composition prepared by incorporating the surfactant by the existing micelle solubilization technology, the S-parameter may be measured/calculated as a value exceeding 1 due to a measurement error. The present invention is not intended to comprise the case of a dispersion composition prepared by incorporating the surfactant by the existing micelle solubilization technology, in which the S-parameter is obtained with a value exceeding 1 due to experimental or measurement error.

On the other hand, when two or more surfactant components exist above the critical micelle concentration, if $S_{surf(i)}$ obtained by Equation 4 below for each type of surfactant is calculated, the $S_{surf}$ value is obtained as the sum of them by Equation 5 below.

<Equation 4>

$$S_{surf(i)} = k_{surf(i)} (C_{surf(i)} - CMC_{surf(i)})$$

wherein $k_{surf(i)}$ is the known molar solubilization capacity ($\kappa$) measured in the dispersion medium for any one type of the surfactants and the target substance, $C_{surf(i)}$ is the concentration of any one type of the surfactant components above the critical micelle concentration, $CMC_{surf(i)}$ is the known critical micelle concentration measured in the dispersion medium of any one type of the surfactant components above the critical micelle concentration.

<Equation 5>

$$S_{surf} = \sum_{i=1}^{m} S_{surf(i)}$$

wherein m is the total number of types of surfactant components above the critical micelle concentration. If the additivity rule between the solubilization capacities of the used surfactants is followed (that is, the content of the target substance solubilized by multiple surfactants is equal to the simple sum of the amounts solubilized by each surfactant), Equation 5 above is effectively applied regardless of whether the plurality of surfactants form pure micelles or mixed micelles in the dispersion medium.

It is known that the above additivity rule is effectively applied between polyoxyethylene glycol (PEG)-based non-ionic surfactants for cyclosporin A used as a target substance in the examples of the present invention (Feng et al, J. Pharmaceutical Sciences, Vol. 107(8), 2018, 2079-2090). Examples of polyoxyethylene glycol (PEG)-based non-ionic surfactants comprise, but are not limited to, Polysorbates (e.g., Tween 20, 40, 60, 80, etc.), polyethoxylated castor oil (e.g., Kolliphor EL, which is Polyoxyl 35 castor oil, or Cremophor EL, Marlowet 40, Emulgin RO 40, etc.), polyethoxylated hydrogenated-castor oil (e.g., Cremophor RH40, etc.), polyethoxylated fatty alcohol(e.g., Brij 30, Brij 35, etc.), polyethoxylated fatty acid(e.g., Myrj 52, Myrj 59, etc.), polyethoxylated hydroxy fatty acid (e.g., Kolliphor HS 15, Solutol HS 15, etc.), Vitamin E TPGS (Vitamin E Tocopheryl Polyethylene Glycol Succinate), Poloxamer (e.g., Poloxamer 407, Lutrol F127, Poloxamer 188, Lutrol F68, etc.). In addition, it is known that the additivity rule between the PEG (polyoxyethylene glycol)-based non-ionic surfactants is effective not only for cyclosporin A used in the examples of the present invention, but also for various active pharmaceutical substances with different chemical structures and physico-chemical physical properties, such as progesterone, ritonavir, butylparaben, etc. (Feng et al, J. Pharmaceutical Sciences, Vol. 107(8), 2018, 2079-2090). That is, the maximum amount ($S_{micelle}$) of the target substance that can be solubilized by micelle solubilization technology can be calculated using known physical properties ($\kappa$, CMC, etc.) measured experimentally for the used surfactants, the target substance, and the dispersion medium, and the content of the target substance that can be contained in the existing composition obtained by solubilizing the target substance by the micelle solubilization technology cannot exceed the above $S_{micelle}$.

If the existing dispersion composition implemented with the micelle solubilization technology contains two or more types of surfactants exceeding the critical micelle concentration, the maximum content of the target substance that can be solubilized by the two or more surfactants is determined by the $S_{micelle}$ value obtained through Equations 1, 4, and 5.

If the dispersion composition implemented by the present invention contains two or more types of surfactants exceeding the critical micelle concentration, the content of the target substance that can be stably contained in the dispersion composition exceeds the $S_{micelle}$ (Equation 1, 4, and 5) values. That is, the S-parameter of the dispersion composition containing two or more types of surfactants implemented by the present invention exceeds 1. For an example, in the dispersion composition implemented by the present invention, the S-parameter value may be 1.05 or more, 1.06 or more, 1.1 or more, 1.2 or more, 1.5 or more, 2 or more, or 3 or more.

Since $S_{micelle}$ is a value calculated using the experimentally measured physical properties of the target substance, the surfactant, and the dispersion medium, the content of the target substance in the dispersion composition implemented with the existing micelle solubilization technology cannot exceed $S_{micelle}$, and thus the S-parameter of the dispersion composition implemented by the existing micelle solubilization technology is 1 or less. However, since the dispersion composition implemented by the present invention has an S-parameter exceeding 1, the present invention can implement a dispersion composition containing a higher content of the target substance beyond the limit that can be solubilized by the existing micelle solubilization technology. That is, when the dispersion composition of the present invention comprises two or more types of surfactants, the S-parameter of the dispersion composition implemented according to the present invention may exceed 1, regardless of the type or total number of surfactants used or the type of the target substance. Expressing this in terms of the surfactant, it means that the present invention can implement a dispersion composition containing the specific content of the target substance even when using a smaller amount of surfactant than the surfactant required to solubilize the specific content of the target substance with the existing micelle solubilization technology.

As described above, with the existing micelle solubilization technology, since the surfactant cannot stably disperse the target substance in the dispersion medium with a content higher than the maximum amount ($S_{micelle}$) that can solubilize the target substance, it is natural in terms of concept definition that the S-parameter for the dispersion composition prepared by incorporating the surfactant by the existing micelle solubilization technology cannot exceed 1.

However, when the S-parameter is experimentally measured for the dispersion composition prepared by incorporating the surfactant by the existing micelle solubilization technology, the S-parameter may be measured/calculated as a value exceeding 1 due to a measurement error. The present invention is not intended to comprise the case of a dispersion composition prepared by incorporating the surfactant by the existing micelle solubilization technology, in which the S-parameter is obtained with a value exceeding 1 due to experimental or measurement error.

As described above, the present invention can implement a dispersion composition containing a higher content of the target substance beyond the limit that can be solubilized by the existing micelle solubilization technology. This is presumed to be due to the fact that molecular clustering or arrangement or conformation of molecules of the target substance in mixed liquor is induced/promoted/caused in a specific direction/shape and then the surface characteristics of the solid target substance formed are changed, due to the physico-chemical interaction described below, which is induced/promoted/caused while contacting the mixed liquor and the medium with a plurality of surfaces in the process of the present invention, which will be described later. It is possible by a plurality of theories and explanations that since the interaction between the changed surface characteristics of the target substance and the added surfactant is changed compared to the existing one, micelles that differ in size or physical properties from the original ones are formed, or the partition coefficient between the micelle-dispersion medium of the target substance becomes different from the existing known technology, or a novel mechanism different from the micelle solubilization mechanism is operated. The foregoing theories and explanations have scientific basis and logical validity, but implementation of the results of the present invention is not limited to being possible only with the foregoing theories or explanations. Other mechanisms that have not been identified may be operated, or a third mechanism may be operated in combination in addition to the above theories or explanations. Whatever the specific molecular unit mechanism is, the present invention can realize a dispersion composition containing a higher content of the target substance beyond the limit that can be solubilized by the existing micelle solubilization technology, and this can be confirmed through numerical comparison with the existing dispersion composition through the aforementioned S-parameter analysis.

In one embodiment of the present invention, the present invention provides a dispersion composition comprising a dispersion medium; and particles containing a target substance,

wherein the dispersion composition comprises at least one type of surfactant having a critical micelle concentration or more,

the dispersion composition does not comprise a solubilizer,

the target substance is cyclosporin A,

if the dispersion composition comprises at least one type of surfactant, the S-parameter of Equation 3 calculated by Equation 1 and Equation 2 satisfies S-parameter > 1, and

if the dispersion composition comprises at least two types of surfactants having a critical micelle concentration or more, $S_{surf(i)}$ obtained by Equation 4 below is calculated for each type of surfactant, and then the $S_{surf}$ value is obtained as the sum of these by Equation 5 below and the S-parameter of Equation 3 obtained by applying the calculated $S_{surf}$ value to Equation 1 above satisfies S-parameter > 1.

<Equation 1>

$$S_{micelle} = S_w + S_{surf}$$

wherein $S_w$ is the concentration corresponding to the saturation solubility of the target substance in the dispersion medium, and $S_{surf}$ is calculated by Equation 2 below.

<Equation 2>

$$S_{surf} = k(C_{surf} - CMC)$$

wherein k is the molar solubilization capacity defined as the number of moles of the target substance that can be dispersed in the dispersion medium by one type of surfactant having the critical micelle concentration of 1 mole or more, and $C_{surf}$ is the molar concentration of the surfactant component in the composition, and CMC is the critical micelle molar concentration of the surfactant in the composition.

<Equation 3>

$$S\text{-parameter} = S_{tot} / S_{micelle}$$

wherein $S_{tot}$ is the total molar content of the target substance contained in the dispersion composition.

<Equation 4>

$$S_{surf(i)} = k_{surf(i)} (C_{surf(i)} - CMC_{surf(i)})$$

wherein $k_{surf(i)}$ is a molar solubilization capacity defined as the number of moles of the target substance that can be dispersed in the dispersion medium by any one type of the surfactant components having the critical micelle concentration of 1 mole or more, $C_{surf(i)}$ is the concentration of any one type of the surfactant components having the critical micelle concentration or more, and $CMC_{surf(i)}$ is the critical micelle concentration in the dispersion medium of any one type of the surfactant components having the critical micelle concentration or more.

<Equation 5>

$$S_{surf} = \sum_{i=1}^{m} S_{surf(i)}$$

wherein m is the total number of types of surfactant components having the critical micelle concentration or more.

When the dispersion composition contains two or more types of surfactants having a critical micelle concentration or more, the type of surfactant may be selected so that the total content of the target substance that can be dispersed corresponds to the sum of the contents of the target substance that can be dispersed for each type of surfactant.

The dispersion composition may comprise the target substance in an amount exceeding a content corresponding to saturation solubility in the dispersion medium.

The dispersed phase particles in the dispersion composition of the present invention may comprise one or more target substances. If the particles comprise multiple types of target substances, the expression stating that "the content of the target substance exceeds the content corresponding to the saturation solubility of the target substance in the dispersion medium" means that at least one substance among the plurality of types of substances is comprised in an amount exceeding the content corresponding to the saturation solubility in the dispersion medium. In addition, the particles may further comprise additives in addition to the target substance.

The particles may be crystalline, amorphous, or a mixture thereof. In one embodiment, the target substance is a drug and the particles are amorphous or crystalline. The particles may be single component or multicomponent. The particles may be single phase or multiphase.

The number average diameter of the particles containing the target substance in the dispersion composition implemented by the present invention has a size of about 100 nm or less.

In one embodiment, the number average diameter may be about 80 nm or less.

In one embodiment, the number average diameter may be about 50 nm or less

In one embodiment, the number average diameter may be about 1 nm to about 20 nm.

In one embodiment, the number average diameter may be about 1 nm to about 10 nm.

In one embodiment, the number average diameter may be about 1 nm to about 5 nm.

The dispersion composition is transparent. If the particle size is small and does not agglomerate or settle, the permeability of the dispersion composition remains high. Since the particles are formed in nanometer unit size, the dispersion composition is formed transparently and permeability is high. The permeability can be measured as permeability or turbidity for a specific wavelength, and the permeability can be also confirmed by visual inspection.

The dispersion composition has excellent dispersion stability. The dispersion composition is stably dispersed while comprising the target substance in an amount exceeding the saturation solubility in the dispersion medium. For example, the stability of the dispersion composition can be confirmed by observing that transparence is maintained over time or that precipitation does not occur, through a visual inspection. As another example, the dispersion stability can be confirmed through the rate of change obtained by measuring the physical properties that are sensitive to particle size, such as permeability or turbidity obtained by optical measurements, or the particle size (Z-avg or mean particle diameter) measured by dynamic light scattering (DLS) over time.

In addition, the dispersion stability can be also measured by the change in the content of the target substance over time. If particles are precipitated for reasons such as agglomeration, the content measured by High Performance Liquid Chromatography (HPLC), which is measured after filtering, is decreased over time. Therefore, the dispersion stability can be confirmed by examining whether the content measured by high performance liquid chromatography after filtering is maintained at the value immediately after production of the composition over time.

For example, the dispersion composition may stably maintain a dispersion state, in which the number average diameter of the particles is 100 nm or less, for about 24 hours or more.

In another example, the dispersion composition may stably maintain a dispersion state, in which the number average diameter of the particles is 100 nm or less, for about 1 week or more.

In another example, the dispersion composition may stably maintain a dispersion state, in which the number average diameter of the particles is 100 nm or less, for about 1 month or more.

In another example, the dispersion composition may stably maintain a dispersion state, in which the number average diameter of the particles is 100 nm or less, for about 3 months or more.

In another example, the dispersion composition may stably maintain a dispersion state, in which the number average diameter of the particles is 100 nm or less, for about 12 months or more.

In one embodiment of the present invention, the present invention provides a solid substance obtained by removing the dispersion medium from the dispersion composition.

In one embodiment of the present invention, the aforementioned dispersion composition may be obtained by applying the solid substance as the target substance.

In one embodiment of the present invention, the present invention provides the solid cyclosporin A obtained by removing the dispersion medium from the dispersion composition.

In one embodiment of the present invention, the aforementioned dispersion composition may be obtained by applying the solid cyclosporin A as a target substance.

In another embodiment of the present invention, the present invention provides a solid substance that can be applied as the target substance. That is, the dispersion composition described above is obtained by applying the solid substance as the target substance.

In one embodiment, the target substance is cyclosporin A, and the solid substance is the solid cyclosporin A.

The solid substance can be stably dispersed in a dispersion medium exceeding the maximum amount ($S_{micelle}$) that can be solubilized by the micelle solubilization technology. In the dispersion composition thus obtained, the S-parameter of Equation 3 satisfies S-parameter > 1 as described above, and a detailed description is the same as that of the aforementioned dispersion composition. This is presumed to be due to the fact that molecular clustering or arrangement or conformation of molecules of the target substance in mixed liquor is induced/promoted/caused in a specific direction/shape and then the surface characteristics of the solid target substance formed are changed, due to the physico-chemical interaction described below, which is induced/promoted/caused while contacting the mixed liquor and the medium with a plurality of surfaces in the process of the present invention.

The solid substance may be a powder.

The dispersion composition according to one embodiment of the present invention has a lower content of surfactant than those of existing technologies, and improves not only the solubility of the target substance but also the permeability, thereby resulting in improved bioavailability and excellent therapeutic efficacy. In addition, by lowering the content of surfactants compared to existing technologies, there is an effect of reducing or preventing problems such as environmental pollution or toxicity/side effects in the body caused by the surfactants. For example, Polyoxyl 35 castor oil (Kolliphor EL or Cremophor EL), which is mainly used as a surfactant in pharmaceutical compositions, is highly toxic. In addition, even when preparing the dispersion of cyclosporin A, Polysorbate 80 (Polysorbate 80 or Tween 80), which is the surfactant, is used, which has the disadvantage of severe eye irritation. The dispersion composition according to one embodiment of the present invention can reduce or avoid the side effects of toxic surfactants because the surfactant content is low and a stable dispersion state is also maintained. In another example, a cosmetic product made using the dispersion composition according to one embodiment of the present invention is a composition that minimizes the content of additives such as surfactant, and can improve cosmetic effects by improving spreadability and penetrability.

The dispersion composition or solid substance (or solid target substance) may be prepared by a preparation method described below.

The preparation method may comprise the steps of preparing a mixed liquor by mixing a target substance and a solvent for preparing mixed liquor; preparing a medium with a plurality of surfaces; contacting the mixed liquor with the medium with a plurality of surfaces.

In one embodiment of the present invention, in the step of contacting the mixed liquor containing the target substance with the medium with a plurality of surfaces, it can induce/facilitate/cause physico-chemical interactions such as shear, confinement effect, and surface effects between the intraparticle pores or interparticle pores of the medium with a plurality of surfaces or the surface inside/outside the pores and the mixed liquor.

The mixed liquor is subjected to a high shear rate while passing between nanometer or micrometer sized intraparticle or interparticle pores of the medium with a plurality of surfaces. Under the condition of such a high shear rate, certain types of molecular clustering or arrangement/alignment or conformation of the molecules of the target substance in the mixed liquor can be induced/facilitated/caused.

In the process of contacting the mixed liquor with the medium with a plurality of surfaces, if the molecules of the target substance are spatially constrained within pores (intraparticle or interparticle) of tens of nanometers or less contained in the medium with a plurality of surfaces (confinement effect), a specific form of molecular clustering or arrangement/alignment or conformation of the molecules of the target substance in the mixed liquor may be induced/facilitated/caused.

When the mixed liquor comes into contact with the surface inside/outside the pores of the medium with a plurality of surface, the molecular clustering or arrangement/alignment or conformation of a specific form of the molecules of the target substance in the mixed liquor can be induced/facilitated/caused depending on the surface characteristics (for example, polarity, hydrophilicity, or type of surface functional groups, etc.) of the medium with a plurality of surfaces.

As the mixed liquor contacts the medium with a plurality of surface, the time for the physico-chemical interaction of the mixed liquor with the medium with a plurality of surfaces is changed depending on the retention time within the medium with a plurality of surfaces, so that the specific form of molecular clustering or arrangement/alignment or conformation of the molecules of the target substance in the mixed liquor may be easily induced/facilitated/caused.

In the process of the present invention, it is assumed that due to the physico-chemical interaction that is induced/facilitated/caused while contacting the mixed liquor and the medium with a plurality of surfaces, and the molecular clustering or arrangement/alignment or conformation of the molecules of the target substance in the mixed liquor is induced/facilitated/caused in a specific direction/shape, and then the surface characteristics of the solid target substance or the particles containing the target substance formed are changed to be more friendly with the dispersion medium. For example, it is assumed that when a target substance that is poorly soluble in water is to be dispersed in water, if the process of the present invention is performed, the specific form of molecular clustering or arrangement/alignment or conformation of the target substances in the mixed liquor is induced/facilitated/caused and then the surface characteristics of the particles of the target substance formed are changed to be more friendly to water, which is a dispersion medium, and thus the dispersibility to water is improved, thereby resulting in a decrease in the amount of surfactant required for solubilization.

The step of contacting the mixed liquor with the medium with a plurality of is a process of inducing/facilitating/causing the above-mentioned various physico-chemical interactions, and comprise a step of continuously passing (flow through) the mixed liquor through the medium with a plurality of surfaces and then collecting the passed mixed liquor or contacting or mixing it with the dispersion medium. In this step, if necessary, the process fluid may be sequentially or parallelly flowed into the medium with a plurality of surfaces together with the mixed liquor. In another embodiment, after impregnation of the mixed liquor into pores in the medium with a plurality of surfaces, a step of contacting the medium with a plurality of surfaces containing the mixed liquor in liquid phase with the dispersion medium (it can be in various ways, such as simple mixing) to release the mixed liquor into the dispersion medium is comprised.

In one embodiment, the flowthrough is obtained by passing the mixed liquor and the process fluid together sequentially or in parallel through the medium with a plurality of surfaces in the form of a packed-bed or membrane or sheet composed of porous or non-porous materials and collecting them directly. All solvents for preparing the mixed liquor and process fluids are removed by lyophilization or normal pressure/reduced pressure/vacuum drying or distillation in the flowthrough to obtain a solid target substance (or the solid substance described above). The separated solid target substance (or solid substance) may be a powder. The desired dispersion composition can be obtained by mixing the obtained solid target substance (or solid substance), the dispersion medium (e.g., water), one or more types of surfactants, and additives (osmotic pressure regulator, thickener, pH buffer, etc.) depending on the purpose and use of the final composition. In particular, the target substance may be poorly soluble in the dispersion medium and the target substance may be supersaturated and thus dispersed in the dispersion medium. In the above example, when the mixed liquor flows through the medium with a plurality of surfaces, if necessary, the retention time or the degree/strength of the physico-chemical interaction may be adjusted by adjusting the pressure (pressure difference between the inlet and the outlet) or temperature during the process.

In the process according to the present invention, when the mixed liquor is contacted by passing through the medium with a plurality of surfaces, the amount of the target substance retained in the medium with a plurality of surfaces is very small, and thus most (more than 95%) of the target substance exits the medium with a plurality of surfaces. The purpose of the process according to the present invention is to apply the physico-chemical interaction to the molecules of the target substance while bringing the mixed liquor into contact with the medium with a plurality of surfaces, and the process according to the present invention is not aimed at inducing/facilitating/causing a thermodynamic phase transition such as solidification (crystallization or amorphization) or precipitation of the target substance within the medium with a plurality of surfaces (which may be in pores). If the target substance is remained by phase transition such as solidification or precipitation in the medium with a plurality of surfaces, it is difficult to implement the result of the present invention (solid target substance (or solid substance) or dispersion composition) or the effect/efficacy according to the present invention. Therefore, in the process according to the present invention, it is important to adjust/control process conditions (e.g., retention time or flow rate) so that the target substance does not remain in the medium with a plurality of surfaces.

In the process according to the present invention, when the mixed liquor is brought into contact by passing through the medium with a plurality of surfaces, the retention time ($t_{ret}$) corresponding to the total time of contact between the mixed liquor and the medium with a plurality of surfaces is calculated by Equation 7 below. In Equation 7 below, q is the Darcy flux, which is the volume of the fluid passing through the unit cross-sectional area of the medium with a plurality of surfaces per unit time, and L is the dimension of the medium with a plurality of surfaces in the direction in which the fluid passes through the medium with a plurality of surfaces (if the medium with a plurality of surfaces is packed-bed, it corresponds to the height of the packed-bed).

<Equation 7>

$$t_{ret} = L/q$$

In the process according to the present invention, in order to induce/facilitate/cause the specific type of molecular clustering or arrangement/alignment or conformation of the molecules of the target substance, the advection rate that causes ordering must dominate the diffusion rate that causes randomization. When expressing this in terms of retention time, if the retention time is too long, since there is sufficient time for diffusion to occur, and thus a specific form of molecular clustering or arrangement/alignment or conformation becomes difficult, it is undesirable if the retention time is too large. On the other hand, if the flow rate is too large and thus the retention time is too small, turbulent flow occurs, which also interferes with molecular clustering or arrangement/alignment or conformation of the target substance. Therefore, if the present invention is implemented by passing the mixed liquor through the medium with a plurality of surfaces, there is a suitable range for the retention time. In order to implement the present invention, it is appropriate to adjust the retention time to be exceeding about 30 sec and less than about 390 sec. On the other hand, according to Equation 7, since the retention time is proportional to the packed-bed height (L), setting the rate (aspect ratio), which is the height (L) of the packed-bed, more precisely the height/diameter of the height of the packed-bed and the diameter of the packed-bed, to be exceeding about 0.01 and less than about 1 helps to obtain the appropriate retention time.

In the flowthrough, it is assumed that the surface characteristics of the solid target substance (or solid substance) obtained by removing all solvents for preparing the mixed liquor or process fluids are changed to be more friendly with the dispersion medium, due to the molecular clustering or arrangement/alignment or conformation of the molecules of the target substance changed due to the physico-chemical interaction that occurs while the mixed liquor passes through the medium with a plurality of surfaces. Accordingly, the solid target substance (or solid substance) according to one embodiment of the present invention can be stably dispersed in a content higher than the maximum amount ($S_{micelle}$) that can be solubilized by the existing micelle solubilization technology in the dispersion medium containing the surfactant. When mixing the target substance with the dispersion medium, one or more types of surfactants, and required additives to produce the dispersion composition, it is assumed that the surface characteristics of the particles containing the target substance are changed to be more friendly with the dispersion medium, thereby improving dispersibility to the dispersion medium, and thus resulting in a decrease in the amount of surfactant required for solubilization. In addition, it is assumed that since the solid target substance (or solid substance) obtained by removing the dispersion medium from the dispersion composition maintains the surface characteristics of these particles even when dispersed in the dispersion medium again, likewise, the dispersibility of the dispersion medium is improved, resulting in a decrease in the amount of surfactant required for solubilization.

In one embodiment of the present invention, the step of contacting the mixed liquor with the medium with a plurality of surfaces as described above is referred to as a unit operation. In one embodiment of the present invention, by performing the unit operation once, it is possible to make the dispersion composition in which the particles comprising the target substance (or the substance) are dispersed. In one embodiment of the present invention, by repeating the unit operation several times under the same process conditions, it is possible to make the dispersion composition in which the particles containing the target substance (or the substance) are dispersed. In one embodiment of the present invention, by performing the unit operation several times while changing/adjusting the process conditions for each unit operation, it is possible to make the dispersion composition in which the particles containing the target substance (or the substance) are dispersed. In this case, the process conditions of each unit operation can be individually or gradually changed/adjusted.

In one embodiment of the present invention, the solid target substance (or solid substance) obtained by removing all solvents for preparing mixed liquor or process fluids in the flowthrough by lyophilization or normal pressure/reduced pressure/vacuum drying or distillation may be mixed with the dispersion medium, one or more types of surfactants, and various additives (osmotic pressure regulator, thickener, pH buffer, etc.) depending on the purpose and use of the final composition to obtain a desired final dispersion composition. In another embodiment, the dispersion medium and one or more types of surfactants may be mixed in the flowthrough, and then the solvent for preparing the mixed liquor or the process fluid may be preferentially separated/removed, and then required additives may be added to obtain a desired final dispersion composition. As a method of preferentially separating/removing only the solvent for preparing mixed liquor or the process fluid, there is a method using a separation technology such as distillation. For example, if the boiling point of the solvent for preparing the mixed liquor or the process fluid is lower than the boiling point of the dispersion medium, the solvent for preparing the mixed liquor or the process fluid may be preferentially separated/removed by heating to a temperature higher than the boiling point of the solvent for preparing the mixed liquor or the process fluid and lower than the boiling point of the dispersion medium. In addition to this, the solvent for preparing the mixed liquor or the process fluid may be preferentially separated/removed by using various separation technologies using the difference in physico-chemical physical properties between the solvent for preparing the mixed liquor or the process fluid and the dispersion medium.

The dispersion composition may include a small amount of the solvent for preparing the mixed liquor or the process fluid remaining without being partially removed. Depending on the application, the content of the solvent for preparing the mixed liquor or the process fluid can be determined. For example, when preparing a composition as a drug, the content can be determined depending on the toxicity of the solvent for preparing the mixed liquor or the process fluid in the body. For example, if the solvent for preparing the mixed liquor or the process fluid is ethanol, the dispersion composition may contain ethanol in an amount less than about 0.5% (w/w) (United States Pharmacopoeia <467> Residual Solvents, December 1, 2020).

The preparation method of the dispersion composition may further include separating and removing the medium with a plurality of surfaces. The medium with a plurality of surfaces can be separated, for example, by filtration using a filter, and in addition, various separation methods such as physical removal, centrifugation, coagulation, precipitation, and electrostatic attraction can be used, but are not limited thereto. The preparation method of the dispersion composition may additionally optionally comprise removing or adding a certain amount of the dispersion medium in the dispersion composition to determine/adjust the final content/concentration of the target substance.

In one embodiment of the present invention,

the present invention provides a method for preparing a solid target substance (or solid substance) comprising the steps of,
preparing a mixed liquor by mixing a target substance and a solvent for preparing mixed liquor;
preparing a medium with a plurality of surfaces;
contacting the mixed liquor with the medium with a plurality of surfaces; and
separating the target substance from the mixed liquor to obtain a solid target substance.

In one embodiment of the present invention,

the present invention provides a method for preparing a solid target substance (or solid substance) comprising the steps of,
preparing a mixed liquor by mixing a target substance and a solvent for preparing mixed liquor;
preparing a medium with a plurality of surfaces in the form of a packed-bed or film or sheet;
passing (flow through) the mixed liquor and the optionally used process fluid through the medium with a plurality of surfaces;
collecting the mixed liquor and optionally used process fluid that has passed through the medium with a plurality of surfaces; and
removing the solvent for preparing the mixed liquor and the process fluid from the collected flowthrough.

In one embodiment of the present invention,

the present invention provides a method for preparing a dispersion composition in which particles, which are a dispersion phase comprising a target substance, are dispersed, comprising the steps of,
preparing a mixed liquor by mixing a target substance and a solvent for preparing mixed liquor;
preparing a medium with a plurality of surfaces in the form of a packed-bed or film or sheet;
passing (flow through) the mixed liquor and the optionally used process fluid through the medium with a plurality of surfaces;
collecting the mixed liquor and optionally used process fluid that has passed through the medium with a plurality of surfaces;
removing the solvent for preparing the mixed liquor and the process fluid from the collected flowthrough to obtain a solid target substance; and
mixing the solid target substance with a dispersion medium, one or more types of surfactants and/or required

EP 4 241 761 A1

additives.

The dispersion composition according to one embodiment of the present invention may be a dispersion composition prepared by obtaining the solid target substance (or solid substance), and then mixing a dispersion medium, one or more types of surfactants, and/or required additives with the solid target substance (or solid substance) as described above.

In one embodiment of the present invention,

the present invention provides a method for preparing a dispersion composition in which particles comprising a target substance, which are a dispersion phase, are dispersed, comprising the steps of,
preparing a mixed liquor by mixing a target substance and a solvent for preparing mixed liquor;
preparing a medium with a plurality of surfaces in the form of a packed-bed or film or sheet;
passing (flow through) the mixed liquor and the optionally used process fluid through the medium with a plurality of surfaces;
collecting the mixed liquor and optionally used process fluid that has passed through the medium with a plurality of surfaces;
mixing a dispersion medium and one or more types of surfactants in the collected flowthrough; and
preferentially removing the solvent for preparing the mixed liquor or the process fluid from the mixture.

In one embodiment of the present invention, the present invention provides pharmaceutical products for animals or humans containing the solid substance (or solid target substance). In one embodiment of the present invention, the present invention provides cosmetic products containing the solid substance (or solid target substance). In one embodiment of the present invention, the present invention provides a food and beverage containing the solid substance (or solid target substance).

In one embodiment of the present invention, the present invention provides a pharmaceutical product for animals or humans containing the dispersion composition. In one embodiment of the present invention, the present invention provides cosmetic products containing the dispersion composition. In one embodiment of the present invention, the present invention provides a food and beverage containing the dispersion composition.

Hereinafter, examples and comparative examples of the present invention are described. The following examples are only examples of the present invention, but the present invention is not limited to the following examples.

**(Example)**

Hereinafter, units expressing content/concentration in this specification, including examples, will be described.

**% (w/v):** Percentage of the mass (g) of the substance to be measured to the volume (mL) of the entire system. For example, mass of target substance (g)/volume of dispersion composition (mL)*100 or mass of solute (g)/volume of solution (mL)*100
**% (w/w)** = Percentage of the mass of the substance being measured to the mass of the entire system. For example, mass of target substance/mass of dispersion composition*100 or mass of target substance/mass of mixed liquor*100
**% (v/v)** = The percentage of the volume of the substance being measured relative to the volume of the entire system. For example, volume of process fluid/volume of solution*100

**Example 1-1 to 1-4: CsA powder preparation**

As a solvent for preparing the mixed liquor, ethanol (hereinafter referred to as 95% v/v ethanol) was prepared in a volume ratio of 95:5 between ethanol and water. 5 g of cyclosporin A (purity 99.1%. TEVA company lot no. 7414004320, hereinafter referred to as CsA), which is a target substance, was dissolved by stirring in 995 g of the 95% v/v ethanol at 500 rpm for 30 minutes using a magnetic bar and a magnetic stirrer to finally obtain a CsA mixed liquor having a concentration of 0.5% w/w. In a 250 mL Erlenmeyer flask, a Buchner funnel (inner diameter 90 mm) was placed, and a 1 um paper filter was placed on the funnel and soaked with 95% v/v ethanol, and then a suction pump was operated with a pressure difference of 0.8 bar to adsorb a paper filter (1 um) to the bottom of the Buchner funnel. 10 g of mesoporous silica powder (ABC Nanotech company, XL-100) was weighed and put into a 250 mL beaker. To the beaker containing the mesoporous silica, 100 g of 95% v/v ethanol was added, and then the mesoporous silica was sufficiently wetted with ethanol by sufficiently stirring using a medicinal spoon so that the mesoporous silica powder and ethanol were well mixed. The mesoporous silica contained in 95% v/v ethanol was slowly poured into a Buchner funnel lined with a paper filter while maintaining a pressure difference of 0.8 bar using a suction pump, to form a mesoporous silica packed-bed with a height of 8 mm and a diameter of 90 mm on the lower part of the Buchner funnel (diameter 90 mm, packed-bed height 8 mm, aspect ratio (= height/diameter) 0.09). When about 1 cm of supernatant liquid remains on the mesoporous silica packed-bed, the suction pump was stopped and the filter filtrate collected in the Erlenmeyer flask was discarded. After that, the 250 mL Erlenmeyer flask was replaced with a 3000 mL Erlenmeyer flask. A 1 um paper filter was laid on the mesoporous silica packed-bed formed in the Buchner funnel, and 1000 g of the previously prepared 0.5% w/w CsA mixed liquor was poured portion-wise into the Buchner funnel several times. As a process fluid, 95% v/v ethanol was prepared in the same way as the solvent for preparing the mixed liquor, and 500 g of this process fluid was additionally poured into a Buchner funnel, and the mixed liquor and the process fluid were passed through the mesoporous silica packed-bed at an average volumetric flow rate of 13.76 ml/min, and collected in a 3000 ml Erlenmeyer flask for a total of 145 minutes. Therefore, the Darcy flux, which is the value obtained by dividing the average volumetric flow rate by the cross-sectional area (diameter of 90 mm) of the packed layer, was 0.216 cm/min, and the retention time in the mesoporous silica packed-bed of the mixed liquor calculated according to Equation 7 was 222 seconds in total (L = 0.8 cm, q = 0.216 cm/min). The flowthrough collected through the mesoporous silica was filtered using a 0.45 um membrane filter. After that, the product was concentrated at 25°C, 150 rpm, 20 mbar for 4 hours using a rotary evaporator (Eyela, OSB-2200), and then the concentrate thus obtained was dried under reduced pressure in a vacuum oven for 12 hours, and the solvent for preparing the mixed liquor and the process fluid were all removed from the flowthrough to finally obtain 4.84 g of CsA powder. The percentage obtained by dividing the amount of CsA (4.84 g) in the solid phase obtained through the process by the amount of CsA (5 g) introduced into the process was defined as the recovery rate (yield), and the value was calculated as 96.8%. That is, it was found that most of CsA (95% or more) passed through the mesoporous silica packed-bed. This process is referred to as Example 1-1. Processes in which the retention time was changed by varying the average volumetric flow rate in the same manner were referred to as Examples 1-2, 1-3, and 1-4, and the main process conditions of each example are listed in Table 1.

Table 1:

| Example | Amount of CsA introduced into the process (g) | Solvent for preparing the mixed liquor | Process fluid | Average volumetric flow rate (ml/min) | Darcy flux (cm/min) | Retention time (sec) - Equation 7 | Amount of CsA obtained after the process (g) | Recovery rate (%) |
|---|---|---|---|---|---|---|---|---|
| 1-1 | 5 | 95% v/v ethanol | 95% v/v ethanol | 13.76 | 0.216 | 222 | 4.84 | 96.8 |
| 1-2 | 5 | 95% v/v ethanol | 95% v/v ethanol | 12.67 | 0.199 | 241 | 4.75 | 95.0 |
| 1-3 | 2 | 95% v/v ethanol | 95% v/v ethanol | 13.15 | 0.207 | 232 | 1.93 | 96.5 |
| 1-4 | 2 | 95% v/v ethanol | 95% v/v ethanol | 12.47 | 0.196 | 245 | 1.90 | 95.0 |

**Examples 2-1 to 2-3: CsA aqueous dispersion composition**

Kolliphor EL (Manufacturer: BASF, lot no. 55573988Q0), Polysorbate 80 (Tween 80, Manufacturer: Croda, lot no.45971), and the CsA powder embodied in Example 1-1 were sequentially added to a 10 mL transparent vial according to the amount shown in Table 2, and stirred at room temperature at 300 rpm for 12 hours to sufficiently mix. 5ml of purified water was initially added to the CsA/Kolliphor EL/Tween80 mixture being stirred, and stirred at 600 rpm for 30 minutes to sufficiently mix, and then additionally added purified water to adjust the total volume to 200 ml, and finally a CsA aqueous dispersion composition in which CsA particles were dispersed in continuous phase water was prepared according to the composition shown in Table 2.

Table 2:

| Composition | CsA aqueous dispersion composition of the present invention | | |
|---|---|---|---|
| | Example 2-1 | Example 2-2 | Example 2-3 |
| CsA (mg) | 40.0 | 40.0 | 40.0 |
| Tween 80 (mg) | 200.0 | 200.0 | 320.0 |
| Kolliphor EL (mg) | 200.0 | 160.0 | 80.0 |
| Purified water (ml) | 199.56 | 199.60 | 199.56 |
| Total volume (ml) | 200 | 200 | 200 |
| Composition of CsA aqueous dispersion composition (% w/v) | CsA: 0.02 (nominal) Tween 80: 0.1 Kolliphor EL: 0.1 | CsA: 0.02 (nominal) Tween 80: 0.1 Kolliphor EL: 0.08 | CsA: 0.02 (nominal) Tween 80: 0.16 Kolliphor EL: 0.04 |

As a Control group to compare/contrast the difference with the existing known technology, a CsA aqueous dispersion composition prepared with commercial CsA powder was prepared by adding each component in the same amount as the CsA aqueous dispersion composition of Examples 2-1, 2-2, and 2-3, according to Table 2 above, using commercial CsA powder (Manufacturer: TEVA, lot no. 7414004320) instead of the CsA powder implemented in Example 1-1 (hereinafter referred to as Control groups 2-1, 2-2, 2-3). It was confirmed that Control groups 2-1, 2-2, and 2-3 prepared with commercially available CsA powder were all observed to form precipitates immediately after preparation, and were observed to be translucent or opaque, and thus the dispersion composition was not realized. In contrast, it was confirmed that the CsA aqueous dispersion compositions of the same composition prepared in Examples 2-1, 2-2, and 2-3 were not observed to form a precipitate after preparation, and were all observed transparently, and thus the dispersion composition was realized.

The CsA aqueous dispersion compositions prepared in Examples 2-1, 2-2, and 2-3 and Control groups 2-1, 2-2, and 2-3 were filtered through a 0.22 um PES (Polyethersulfone) filter to obtain filter filtrates. The filter filtrates were subjected to HPLC analysis under the following conditions, and the CsA content/concentration was measured and shown in Table 3, respectively. Since the content in the filter filtrate corresponds to the amount of CsA stably dispersed in water, excluding the amount of CsA precipitated at or immediately after preparation, the CsA content/concentrations shown in Table 3 correspond to the actual content/concentrations of CsA stably dispersed in the CsA aqueous dispersion compositions prepared with Example 2-1,2-2,2-3 and Control groups 2-1, 2-2, 2-3.

Instrument: Agilent 1260 Infinity II
Column: Hypersil ODF (4.6 X 250mm, 3gm)
Column temperature: 50 °C
Flow rate: 1 mL/min
Detector: Ultraviolet absorbance photometer (measurement wavelength: 210 nm)
Injection volume: 40 $\mu$L

As shown in Table 3 below, it can be seen that the actual contents of CsA in the CsA aqueous dispersion compositions prepared in Example 2-1, 2-2, 2-3 were all implemented within +/-50 of the nominal content of 0.02% w/v in Table 2. In contrast, it was confirmed that the actual content of CsA in CsA aqueous dispersion compositions prepared with control groups 2-1, 2-2, and 2-3 were all less than the nominal content of 0.02% w/v, and thus a dispersion composition in which 0.02% w/v CsA was dispersed was not implemented.

Table 3:

| HPLC analysis | CsA aqueous dispersion composition of present invention | | | CsA aqueous dispersion composition implemented with known technology | | |
|---|---|---|---|---|---|---|
| | Example 2-1 | Example 2-2 | Example 2-3 | Control group 2-1 | Control group 2-2 | Control group 2-3 |
| CsA content/ concentration (% w/v) | 0.02 | 0.019 | 0.02 | 0.014 | 0.016 | 0.016 |

**<S-parameter evaluation of CsA aqueous dispersion composition>**

Table 4 shows CsA physical properties required for calculation of S-parameters of CsA aqueous dispersion composition, the used surfactant Tween 80 and Kolliphor EL physical properties, and the molar solubilization capacity measured in the surfactant for CsA (Feng et al, J. Pharmaceutical Sciences, Vol. 107(8), 2018, 2079-2090), etc.

Table 4:

| | CsA | Tween 80 | Kolliphor EL |
|---|---|---|---|
| Molecular weight (g/mol) | 1202.6 | 1310 | 2500 |
| Saturation solubility in water, Sw (% w/v) | 0.0027 | n/a | n/a |
| CMC (% w/v) | n/a | 0.0018 | 0.009 |
| Molar solubilization capacity, k | n/a | 0.0806 | 0.1800 |

By inputting the physical properties of Table 4, the surfactant content of Table 2 and the actual content of CsA in Table 3 into Equations 1, 3, 4 and 5, the S-parameter of the CsA aqueous dispersion composition implemented in Examples 2-1, 2-2, and 2-3 was calculated, and Table 5 lists each process and final results. (Since the molar solubilization capacity is the ratio of the number of moles of CsA and the number of moles of surfactant used, when calculating Equation 4, the % w/v composition of the surfactant is converted into the number of moles as the molecular weight of the surfactant and it is input. In addition, since the calculated value is the number of moles of CsA, it is converted into CsA % w/v using the molecular weight of CsA again to obtain $S_{surf}$. The contents/compositions in Tables 5 and 6 are all % w/v obtained through these conversion processes).

Table 5:

| Content/Composition (% w/v) S-parameter (No degree) | CsA aqueous dispersion composition of the present invention | | |
|---|---|---|---|
| | Example 2-1 | Example 2-2 | Example 2-3 |
| CsA, $S_{tot}$ (% w/v) | 0.02 | 0.019 | 0.02 |
| Tween 80, $C_{surf}$ (Tween 80), % w/v | 0.1 | 0.1 | 0.16 |
| Kolliphor EL, $C_{surf}$ (Kolliphor EL), % w/v | 0.1 | 0.08 | 0.04 |
| $S_{surf}$ (Tween 80) - Equation 4 | 0.00726 | 0.00726 | 0.0117 |
| $S_{surf}$ (Kolliphor EL) - Equation 4 | 0.00788 | 0.00615 | 0.00268 |
| $S_{surf} = S_{surf}$ (Tween 80) $+ S_{surf}$ (Kolliphor EL) - Equation 5 | 0.01514 | 0.01341 | 0.01439 |
| $S_{micelle} = S_w + S_{surf}$ - Equation 1 | 0.0178 | 0.016 | 0.0171 |
| S-parameter (=$S_{tot}/ S_{micelle}$) - Equation 3 | 1.12 | 1.18 | 1.17 |

In the same way, by inputting the physical properties of Table 4, the surfactant content of Table 2, and the actual CsA content of Table 3 into Equation 1, 3, 4 and 5, S-parameters of CsA aqueous dispersion compositions implemented with control groups 2-1, 2-2, and 2-3 were calculated, and each process and final result are described in Table 6.

Table 6:

| Content/Composition (% w/v) S-parameter (No degree) | CsA aqueous dispersion composition of known technology | | |
|---|---|---|---|
| | Control group 2-1 | Control group 2-2 | Control group 2-3 |
| CsA, $S_{tot}$ (% w/v) | 0.014 | 0.016 | 0.016 |
| Tween 80, $C_{surf}$ (Tween 80), % w/v | 0.1 | 0.1 | 0.16 |
| Kolliphor EL, $C_{surf}$ (Kolliphor EL), % w/v | 0.1 | 0.08 | 0.04 |
| $S_{surf}$ (Tween 80) - Equation 4 | 0.00726 | 0.00726 | 0.0117 |
| $S_{surf}$ (Kolliphor EL) - Equation 4 | 0.00788 | 0.00615 | 0.00268 |
| $S_{surf} = S_{surf}$ (Tween 80) + $S_{surf}$ (Kolliphor EL) - Equation 5 | 0.01514 | 0.01341 | 0.01439 |
| $S_{micelle} = S_w + S_{surf}$ - Equation 1 | 0.0178 | 0.016 | 0.0171 |
| S-parameter (=$S_{tot}$/ $S_{micelle}$) - Equation 3 | 0.79 | 1.0 | 0.94 |

As shown in Table 5, the S-parameters of the CsA dispersion compositions implemented in Example 2-1, 2-2 and 2-3 are 1.12, 1.18 and 1.17, respectively, all exceeding 1. This means that they exceed the existing limit ($S_{micelle}$) amount of CsA in the CsA dispersion compositions implemented in Example 2-1, 2-2 and 2-3 that can be solubilized with the two types of surfactants (Tween 80 and Kolliphor EL) by 12%, 18%, and 17%, respectively. In contrast, as shown in Table 6, all of the S-parameters of the dispersion compositions of Control groups 2-1, 2-2, and 2-3 implemented by known technologies are 1 or less. That is, it can be seen that even if it is prepared by adding the same amount of surfactant and the same amount of CsA, the known technology does not contain the target amount of CsA in the dispersion composition because the amount of CsA in excess of $S_{micelle}$ is precipitated. Therefore, by using the present invention, it is possible to disperse a high content of CsA, which cannot be realized by the existing known technology. Expressing in terms of surfactant, this means that a smaller amount of surfactant is required than the known technology in dispersing the same content (0.02%) of CsA.

**Experimental Example 1-1: Dispersion stability of CsA aqueous dispersion composition: visual inspection**

In order to check the stability of the CsA aqueous dispersion compositions prepared in Example 2-1, 2-2 and 2-3, after preparation, these were stored for 2 to 4 weeks at 25 $\pm$ 2 °C and relative humidity of 60 $\pm$ 5%, and visual inspection was performed for precipitation or change in transparency over time, and Table 7 shows the results of visual inspection according to the elapsed time after preparation. As shown in Table 7, it was confirmed that all of the CsA aqueous dispersion compositions prepared in Examples 2-1, 2-2 and 2-3 maintain transparency without precipitation for 2 to 4 weeks. Therefore, it can be seen that during this period, in the CsA aqueous dispersion composition prepared in Examples 2-1, 2-2, and 2-3, CsA was stably dispersed in water without precipitation of CsA or aggregation/agglomeration of particles.

Table 7:

| Elapsed time since preparation | CsA aqueous dispersion composition of the present invention | | | | | |
|---|---|---|---|---|---|---|
| | Example 2-1 | | Example 2-2 | | Example 2-3 | |
| | Precipita tion | Condition | Precipita tion | Condition | Precipita tion | Condition |
| 1 week | no | transparent | no | transparent | no | transparent |
| 2 weeks | no | transparent | no | transparent | no | transparent |
| 3 weeks | no | transparent | no | transparent | | |
| 4 weeks | no | transparent | no | transparent | | |

**Experimental Example 1-2: Dispersion stability of CsA aqueous dispersion composition: HPLC content**

In order to check the stability of the CsA aqueous dispersion compositions prepared in Examples 2-1, 2-2, and 2-3, after preparation, these were stored for 2 to 4 weeks at 25 $\pm$ 2 °C and relative humidity of 60 $\pm$ 5%, and the content of CsA was measured over time by HPLC. Immediately after preparation, after 1 week, 2 weeks, and 4 weeks, the CsA aqueous dispersion compositions prepared in Example 2-1, 2-2 and 2-3 were filtered with a 0.22 um PES (polyethersulfone) filter, and then the filter filtrate was subjected to HPLC analysis under the following conditions to measure the rate of change in the content of CsA over time (calculation of the content change rate over time by setting the HPLC content as 100% immediately after preparation), and the results are listed in Table 8.

Instrument: Agilent 1260 Infinity II
Column: Hypersil ODF (4.6 X 250mm, 3gm)
Column temperature: 50 °C
Flow rate: 1 mL/min
Detector: Ultraviolet absorbance photometer (measurement wavelength: 210 nm)
Injection volume: 40 $\mu$L

Table 8:

| Elapsed time since preparation | Rate of change in content of CsA aqueous dispersion composition of the present invention | | |
|---|---|---|---|
| | Example 2-1 | Example 2-2 | Example 2-3 |
| Immediately after preparation | 100% | 100% | 100% |
| 1 week | 100.5% | 100.2% | 98.4% |
| 2 weeks | 100.8% | 100.7% | 100.7% |
| 4 weeks | 100.4% | 99.9% | |

As shown in Table 8, the contents of the CsA aqueous dispersion compositions prepared in Examples 2-1, 2-2 and 2-3 are maintained even after 2 to 4 weeks. Therefore, it can be seen that during these periods, CsA was not precipitated and was stably dispersed in the CsA aqueous dispersion compositions prepared in Examples 2-1, 2-2 and 2-3.

**Example 2-4: CsA aqueous dispersion composition**

As a solvent for preparing the mixed liquor, ethanol (hereinafter referred to as 95% v/v ethanol) was prepared in a volume ratio of 95: 5 between ethanol and water. 0.06 g of cyclosporin A (purity: 99.1%, Manufacturer: TEVA company, lot no. 7414004320, hereinafter referred to as CsA), which is a target substance, was dissolved by stirring in 29.94 g of the 95% v/v ethanol at 500 rpm for 30 minutes using a magnetic bar and a magnetic stirrer to finally obtain a CsA mixed liquor having a concentration of 0.2% w/w. In a 250 mL Erlenmeyer flask, a Buchner funnel (inner diameter of 90 mm) was placed, and a 1 um paper filter was placed on the funnel and soaked with 95% v/v ethanol, and then a suction pump was operated with a pressure difference of 0.8 bar to adsorb a paper filter (1 um) to the bottom of the Buchner funnel. 10 g of mesoporous silica powder (ABC Nanotech company, XL-100) was weighed and put into a 250 mL beaker. 100 g of 95% v/v ethanol was added to the beaker containing the mesoporous silica, and then the mesoporous silica was sufficiently wetted with ethanol by sufficiently stirring using a medicinal spoon so that the mesoporous silica powder and ethanol were well mixed. The mesoporous silica contained in 95% v/v ethanol was slowly poured into a Buchner funnel lined with a paper filter while maintaining a pressure difference of 0.8 bar using a suction pump, to form a mesoporous silica packed-bed with a height of 8 mm and a diameter of 90 mm on the lower part of the Buchner funnel (diameter 90 mm, packed-bed height 8 mm, aspect ratio (= height/diameter) 0.09). When about 1 cm of supernatant liquid remains on the mesoporous silica packed-bed, the suction pump was stopped and the filter filtrate passed through the paper filter and collected in the Erlenmeyer flask was discarded. After that, the 250 mL Erlenmeyer flask was replaced with another 250 mL Erlenmeyer flask. A 1 um paper filter was laid on the mesoporous silica packed-bed formed in the Buchner funnel, and 30 g of the previously prepared 0.2% w/w CsA mixed liquor was poured portion-wise into the Buchner funnel several times. As a process fluid, 95% v/v ethanol was prepared in the same way as the solvent for preparing the mixed liquor, and 120 g of this process fluid was mixed with 80 g of purified water, and 200 g of the mixed liquor was additionally poured into a Buchner funnel. 205 g of flowthrough collected through mesoporous silica was filtered using a 0.45 um membrane filter.

A 250 mL concentration flask was prepared and a magnetic bar was placed, and 10 g of the flowthrough was aliquoted, 6 mg of each of Polysorbate 80 (Tween 80, Manufacturer: TCI, XHLAA-GM) and Polyoxyl-35 Castor oil (Manufacturer: ACROS, lot no. A0403500) was added to 10 g of 95% v/v ethanol, and then mixed using a magnetic stirrer for 10 minutes to mix well. After 10 minutes, 40 mL of purified water was added to a 250 mL concentration flask, and the mixture was stirred at 500 rpm for 30 minutes with a magnetic stirrer. Ethanol was selectively removed from the mixed solution using a rotary evaporator (Eyela, OSB-2200) at 25 °C, 150 rpm, and 20 mbar for 2 hours and 20 minutes, and a part of the purified water was distilled to finally obtain 15.8 mL of CsA aqueous dispersion composition. The obtained liquid was filtered through a 0.45 um (Manufacturer: FUTECS, PVDF) syringe filter and a 0.22 um (Manufacturer: FUTECS, PTFE) filter to obtain a filter filtrate. The obtained filter filtrate was subjected to HPLC analysis under the following conditions to measure CsA content/concentration, and the results are shown in Table 9.

HPLC Instrument: Waters
Model name: e2695
Column: RP C18 (250x4.6mm) mean particle size 5 um
Column temperature: 65°C
Flow rate: 1 mL/min
Detector: Ultraviolet absorbance photometer (measurement wavelength: 204 nm)
Injection volume: 10μL

**Example 2-5: CsA aqueous dispersion composition**

As a solvent for preparing the mixed liquor, ethanol (hereinafter referred to as 95% v/v ethanol) was prepared in a volume ratio of 95: 5 between ethanol and water. 0.06 g of cyclosporin A (purity 99.1%, Manufacturer: TEVA company, lot no. 7414004320, hereinafter referred to as CsA), which is a target substance, was dissolved by stirring in 29.94 g of the 95% v/v ethanol at 500 rpm for 30 minutes using a magnetic bar and a magnetic stirrer to finally obtain a CsA mixed liquor having a concentration of 0.2% w/w. In a 250 mL Erlenmeyer flask, a Buchner funnel (inner diameter of 90 mm) was placed, and a 1 um paper filter was placed on the funnel and soaked with 95% v/v ethanol, and then a suction pump was operated with a pressure differential of 0.8 bar to adsorb a paper filter (1 um) to the bottom of the Buchner funnel. 10 g of mesoporous silica powder (ABC Nanotech company, XL-100) was weighed and put into a 250 mL beaker. 100 g of 95% v/v ethanol was added to the beaker containing the mesoporous silica, and then the mesoporous silica was sufficiently wetted with ethanol by sufficiently stirring using a medicinal spoon so that the mesoporous silica powder and ethanol were well mixed. The mesoporous silica contained in 95% v/v ethanol was slowly poured into a Buchner funnel lined with a paper filter while maintaining a pressure difference of 0.8 bar using a suction pump, to form a mesoporous silica packed-bed with a height of 8 mm and a diameter of 90 mm on the lower part of the Buchner funnel (diameter of 90 mm, packed-bed height of 8 mm, aspect ratio (= height/diameter) 0.09). When about 1 cm of supernatant liquid remains on the mesoporous silica packed-bed, the suction pump was stopped and the filter filtrate passed through the paper filter and collected in the Erlenmeyer flask was discarded. After that, the 250 mL Erlenmeyer flask was replaced with new 250 mL Erlenmeyer flask. A 1 um paper filter was laid on the mesoporous silica packed-bed formed in the Buchner funnel, and 30 g of the previously prepared 0.2% w/w CsA mixed liquor was poured portion-wise into the Buchner funnel several times. As a process fluid, 95% v/v ethanol was prepared in the same way as the solvent for preparing the mixed liquor, and 120 g of this process fluid was mixed with 80 g of purified water, 200 g of the mixed liquor was additionally poured into a Buchner funnel. 205 g of flowthrough collected through mesoporous silica was filtered using a 0.45 um membrane filter.

A 250 mL concentration flask was prepared and a magnetic bar was placed, and 10 g of the flowthrough was aliquoted, 3 mg of Polysorbate 80 (Tween 80, Manufacturer: TCI, XHLAA-GM) and 9 mg of Polyoxyl-35 Castor oil (Manufacturer: ACROS, lot no. A0403500) was added to 10 g of 95% v/v ethanol, and then mixed using a magnetic stirrer for 10 minutes to mix well. After 10 minutes, 40 mL of purified water was added to a 250 mL concentration flask, and the mixture was stirred at 500 rpm for 30 minutes with a magnetic stirrer. Ethanol was selectively removed from the mixed solution using a rotary evaporator (Eyela, OSB-2200) at 25 °C, 150 rpm, and 20 mbar for 1 hours and 20 minutes, and a part of the purified water was distilled to finally obtain 15.6 mL of the CsA aqueous dispersion composition. The obtained liquid was filtered through a 0.45 um (Manufacturer: FUTECS, PVDF) syringe filter and a 0.22 um (Manufacturer: FUTECS, PTFE) filter to obtain a filter filtrate. The obtained filter filtrate was subjected to HPLC analysis under the following conditions to measure CsA content/concentration, and the results are shown in Table 9.

HPLC Instrument: Waters
Model name: e2695

Column: RP C18 (250x4.6mm) mean particle size 5 um
Column temperature: 65°C
Flow rate: 1 mL/min
Detector: Ultraviolet absorbance photometer (measurement wavelength: 204 nm)
Injection volume: 10pL

The composition of the final CsA aqueous dispersion compositions of Examples 2-4 and 2-5 is shown in Table 9.

Table 9:

| Composition | CsA aqueous dispersion composition | |
|---|---|---|
| | Example 2-4 | Example 2-5 |
| Composition (% w/v) | CsA: 0.015<br>Tween 80: 0.038 | CsA: 0.018<br>Tween 80: 0.019 |
| | Polyoxyl 35 Castor oil: 0.038 | Polyoxyl 35 Castor oil: 0.058 |

By inputting the physical properties of Table 4, the content of surfactant and the actual content of CsA in Table 9 into Equation 1, 3, 4, and 5, the S-parameters of the CsA aqueous dispersion compositions implemented in Examples 2-4 and 2-5 were calculated, and each process and final results are listed in Table 10 (Since the molar solubilization capacity is the ratio of the number of moles of CsA and the number of moles of surfactant used, when calculating Equation 4, the % w/v composition of the surfactant is converted into the number of moles as the molecular weight of the surfactant and it is input. In addition, since the calculated value is the number of moles of CsA, it is converted into CsA % w/v using the molecular weight of CsA again to obtain $S_{surf}$. The content/compositions in Table 10 are all % w/v obtained through this conversion process).

Table 10:

| Content/Composition (%w/v) S-parameter (No degree) | CsA aqueous dispersion composition | |
|---|---|---|
| | Example 2-4 | Example 2-5 |
| CsA, $S_{tot}$ (% w/v) | 0.015 | 0.018 |
| Tween 80, $C_{surf}$ (Tween 80), % w/v | 0.038 | 0.019 |
| Polyoxyl-35 Castor oil, $C_{surf}$ (Polyoxyl-35 Castor oil), % w/v | 0.038 | 0.058 |
| $S_{surf}$ (Tween 80) - Equation 4 | 0.00268 | 0.00127 |
| $S_{surf}$ (Polyoxyl-35 Castor oil) - Equation 4 | 0.00251 | 0.00424 |
| $S_{surf} = S_{surf}$ (Tween 80) + $S_{surf}$ (Polyoxyl-35 Castor oil) - Equation 5 | 0.00519 | 0.00551 |
| $S_{micelle} = S_w + S_{surf}$ - Equation 1 | 0.0079 | 0.0082 |
| S-parameter (=$S_{tot}/S_{micelle}$) - Equation 3 | 1.90 | 2.19 |

As shown in Table 10, the S-parameters of the CsA aqueous dispersion compositions implemented in Examples 2-4 and 2-5 were 1.90 and 2.19, respectively, all exceeding 1. This means that they exceed the existing limit ($S_{micelle}$) amount of CsA in the CsA dispersion compositions implemented in Example 2-4 and 2-5 that can be solubilized with the two types of surfactants (Tween 80 and Polyoxyl-35 Castor oil) by 90% and 119%, respectively.

Although the present invention has been described as above, the present invention is not limited by the examples disclosed herein. It is obvious that various modifications can be made by a person skilled in the art within the scope of the technical spirit of the present invention. In addition, even if the working effect according to the configuration of the present invention was not explicitly described and explained while explaining the example of the present invention above, it is natural that the predictable effect of the configuration should also be recognized.

**Claims**

1. A dispersion composition comprising:

   a dispersion medium; and particles comprising a target substance,
   wherein the dispersion composition comprises at least one type of surfactant having a critical micelle concentration or more,
   the dispersion composition does not comprise a solubilizer,
   the target substance is cyclosporin A,
   if the dispersion composition comprises at least one type of surfactant, the S-parameter of Equation 3 calculated by Equation 1 and Equation 2 satisfies S-parameter > 1, and
   if the dispersion composition comprises at least two types of surfactants having a critical micelle concentration or more, $S_{surf(i)}$ obtained by Equation 4 below is calculated for each type of surfactant, and then the $S_{surf}$ value is obtained as the sum of these by Equation 5 below and the S-parameter of Equation 3 obtained by applying the calculated $S_{surf}$ value to Equation 1 above satisfies S-parameter > 1:

$$<Equation\ 1>$$
$$S_{micelle} = S_w + S_{surf}$$

   wherein $S_w$ is the concentration corresponding to the saturation solubility of the target substance in the dispersion medium, and $S_{surf}$ is calculated by Equation 2 below,

$$<Equation\ 2>$$
$$S_{surf} = k(C_{surf} - CMC)$$

   wherein k is the molar solubilization capacity defined as the number of moles of the target substance that can be dispersed in the dispersion medium by one type of surfactant having the critical micelle concentration of 1 mole or more, and $C_{surf}$ is the molar concentration of the surfactant component in the composition, and CMC is the critical micelle molar concentration of the surfactant in the composition,

$$<Equation\ 3>$$
$$S\text{-}parameter = S_{tot} / S_{micelle}$$

   wherein $S_{tot}$ is the total molar content of the target substance contained in the dispersion composition,

$$<Equation\ 4>$$
$$S_{surf(i)} = k_{surf(i)} (C_{surf(i)} - CMC_{surf(i)})$$

   wherein $k_{surf(i)}$ is a molar solubilization capacity defined as the number of moles of the target substance that can be dispersed in the dispersion medium by any one type of the surfactant components having the critical micelle concentration of 1 mole or more, $C_{surf(i)}$ is the concentration of any one of the surfactant components having the critical micelle concentration or more, and $CMC_{surf(i)}$ is the critical micelle concentration in the dispersion medium of any one type of the surfactant components having the critical micelle concentration or more, and

$$<Equation\ 5>$$

$$S_{surf} = \sum_{i=1}^{m} S_{surf(i)}$$

wherein m is the total number of types of surfactant components having the critical micelle concentration or more.

2. The dispersion composition according to claim 1, wherein the dispersion composition comprises the cyclosporin A in an amount exceeding the content corresponding to saturation solubility in the dispersion medium.

3. The dispersion composition according to claim 1, wherein if the two or more types of surfactants having the critical micelle concentration or more are comprised, the type of surfactants is selected so that the total content of dispersible cyclosporin A conforms to the sum of the content of dispersible cyclosporin A for each type of surfactant.

4. The dispersion composition according to claim 1, wherein the number average diameter of the particles has a size of 100 nm or less.

5. The dispersion composition according to claim 1, wherein the number average diameter of the particles has a size of 50 nm or less.

6. A pharmaceutical product for animal or human use comprising the dispersion composition according to claim 1.

7. A cosmetic product comprising the dispersion composition according to claim 1.

8. A food and beverage comprising the dispersion composition according to claim 1.

9. A solid cyclosporin A, in which the dispersion composition according to claim 1 is obtained by applying the cyclosporin A as the target substance of claim 1.

10. A pharmaceutical product for animal or human use comprising the solid cyclosporin A according to claim 9.

11. A cosmetic product comprising the solid cyclosporin A according to claim 9.

12. A food and beverage comprising the solid cyclosporin A according to claim 9.

## INTERNATIONAL SEARCH REPORT

| International application No. |
|---|
| **PCT/KR2021/015913** |

### A. CLASSIFICATION OF SUBJECT MATTER

**A61K 9/10**(2006.01)i; **A61K 47/02**(2006.01)i; **A61K 47/40**(2006.01)i; **A61K 47/38**(2006.01)i; **A61K 47/18**(2006.01)i; **A61K 47/12**(2006.01)i; **A61K 47/26**(2006.01)i; **A61K 31/404**(2006.01)i; **A61K 31/4439**(2006.01)i; **A61K 31/506**(2006.01)i

According to International Patent Classification (IPC) or to both national classification and IPC

### B. FIELDS SEARCHED

Minimum documentation searched (classification system followed by classification symbols)

A61K 9/10(2006.01); A61K 38/12(2006.01); A61K 47/26(2006.01); A61K 9/107(2006.01); A61K 9/14(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 분산 (dispersion), 임계미셀농도 (critical micelle concentration), 가용화제 (solubilizing agent), 계면활성제 (surfactant), 사이클로스포린 A (cyclosporin A)

### C. DOCUMENTS CONSIDERED TO BE RELEVANT

| Category* | Citation of document, with indication, where appropriate, of the relevant passages | Relevant to claim No. |
|---|---|---|
| A | LODHA, A. et al. Synthesis of mesoporous silica nanoparticles and drug loading of poorly water soluble drug cyclosporin A. Journal of pharmacy & bioallied sciences. 2012, 4(Suppl 1), pp. S92-S94. See abstract; and page S93. | 1-12 |
| A | KR 10-1996-0000247 A (YUHAN CORPORATION) 25 January 1996 (1996-01-25) See entire document. | 1-12 |
| A | JP 2007-126423 A (MITSUBISHI CHEMICALS CORP.) 24 May 2007 (2007-05-24) See entire document. | 1-12 |
| A | SEEDHER, N. et al. Micellar solubilization of some poorly soluble antidiabetic drugs: a technical note. AAPS PharmSciTech. 2008, vol. 9, no. 2, pp. 431-436. See entire document. | 1-12 |
| A | WO 2018-152334 A1 (MOLECULAR INFUSIONS, LLC) 23 August 2018 (2018-08-23) See entire document. | 1-12 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **10 February 2022** | **10 February 2022** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
Information on patent family members

International application No.

**PCT/KR2021/015913**

| Patent document cited in search report | | | Publication date (day/month/year) | Patent family member(s) | | | Publication date (day/month/year) |
|---|---|---|---|---|---|---|---|
| KR | 10-1996-0000247 | A | 25 January 1996 | AT | 183398 | T | 15 September 1999 |
| | | | | AU | 2577295 | A | 21 December 1995 |
| | | | | CN | 1148224 | C | 05 May 2004 |
| | | | | CN | 1149834 | A | 14 May 1997 |
| | | | | DE | 69511539 | T2 | 30 March 2000 |
| | | | | DK | 0756489 | T3 | 06 December 1999 |
| | | | | EP | 0756489 | A1 | 05 February 1997 |
| | | | | EP | 0756489 | B1 | 18 August 1999 |
| | | | | ES | 2137516 | T3 | 16 December 1999 |
| | | | | GR | 3031568 | T3 | 31 January 2000 |
| | | | | JP | 09-510733 | A | 28 October 1997 |
| | | | | JP | 2936209 | B2 | 23 August 1999 |
| | | | | KR | 10-0163212 | B1 | 01 December 1998 |
| | | | | RU | 2119351 | C1 | 27 September 1998 |
| | | | | US | 5739105 | A | 14 April 1998 |
| | | | | WO | 95-32726 | A1 | 07 December 1995 |
| JP | 2007-126423 | A | 24 May 2007 | JP | 5113328 | B2 | 09 January 2013 |
| WO | 2018-152334 | A1 | 23 August 2018 | AU | 2018-221739 | A1 | 29 August 2019 |
| | | | | CA | 3053158 | A1 | 23 August 2018 |
| | | | | CN | 110636834 | A | 31 December 2019 |
| | | | | CO | 2019009986 | A2 | 30 September 2019 |
| | | | | EP | 3582755 | A1 | 25 December 2019 |
| | | | | JP | 2020-509081 | A | 26 March 2020 |
| | | | | MX | 2019009642 | A | 11 November 2019 |
| | | | | US | 2020-0037638 | A1 | 06 February 2020 |
| | | | | US | 2020-0246404 | A1 | 06 August 2020 |
| | | | | WO | 2019-036243 | A1 | 21 February 2019 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4839342 A
- US 5051402 A
- US 2014020662 A1
- US 2014005785 A
- US A1 A
- EP 2049079 B1

### Non-patent literature cited in the description

- **REINTJES, T.** Solubility enhancement with BASF Pharma polymers: Solubilizer Compendium. *BASF,* 2021, 9-10
- **O. WOLK et al.** United States Pharmacopeia (USP) Solubility Criteria. *Drug Design, Development and Therapy,* 2014, vol. 8, 1563-1575
- **M.J. ROSEN ; J.T. KUNJAPPU.** Surfactants and Interfacial Phenomena. Wiley, 2012
- **M. J. ROSEN ; J.T. KUNJAPPU.** Surfactants and Interfacial Phenomena. Wiley, 2012, 141-143, 155
- **RANGEL-YAGUI CO ; PESSOA A JR ; TAVARES LC.** *J Pharm Pharm Sci.,* 2005, vol. 8 (2), 147-65
- **FENG et al.** *J. Pharmaceutical Sciences,* 2018, vol. 107 (8), 2079-2090